# EUROPEAN PATENT SPECIFICATION

(11) **EP 2 091 942 B1**
(45) Date of publication and mention of the grant of the patent: **17.02.2010**
(21) Application number: 07821116.6
(22) Date of filing: 10.10.2007
(51) Int. Cl.: C07D 401/12, C07D 241/20, A61K 31/501, A61P 25/00

(54) **SUBSTITUTED PYRAZINONE DERIVATIVES FOR USE AS A MEDICINE**
SUBSTITUIERTE PYRAZINONDERIVATE ZUR VERWENDUNG ALS ARZNEIMITTEL
DÉRIVÉS DE PYRAZINONE SUBSTITUÉS POUR UNE UTILISATION EN TANT QUE MÉDICAMENT

(30) Priority: 12.10.2006 EP 06122173
(43) Date of publication of application: 26.08.2009
(73) Proprietor: Janssen Pharmaceutica NV, 2340 Beerse (BE)
(72) Inventor: ANDRÉS-GIL, José Ignacio, E-45007 Toledo (ES); ALCÁZAR-VACA, Manuel Jesús, E-45007 Toledo (ES); LINARES DE LA MORENA, Maria Lourdes, 45007 Toledo (ES); MARTINEZ GONZALEZ, Sonia, 45007 Toledo (ES); OYARZABAL SANTAMARINA, Julen, 45280 Olias del Rey (Toledo) (ES); PASTOR-FERNÁNDEZ, Joaquin, 45007 Toledo (ES); VEGA-RAMIRO, Juan Antonio, 45007 Toledo (ES); DELGADO-JIMÉNEZ, Francisca, 45007 Toledo (ES); DRINKENBURG, Wilhelmus Helena Ignatius Maria, 2340 Beerse (BE)
(74) Representative: Quaghebeur, Luc
(86) International application number: PCT/EP2007/060748
(87) International publication number: WO 2008/043775

(56) References cited:
- WO-A-2004/009586
- HIEBLE J P ET AL: "ALPHA AND BETA-ADRENOCEPTORS: FROM THE GENE TO THE CLINIC. I MOLECULAR BIOLOGY AND ADRENOCEPTOR SUBCLASSIFICATION" JOURNAL OF MEDICINAL CHEMISTRY, AMERICAN CHEMICAL SOCIETY. WASHINGTON, US, vol. 38, no. 18, 1 September 1995 (1995-09-01), pages 3415-3444, XP002004720 ISSN: 0022-2623
- J. W. KABLE ET AL.: "In vivo gene modification elucidates subtype-specific functions of alpha2-adrenergic receptors" J. PHARMACOL. EXP. THER., vol. 293, no. 1, 2000, pages 1-7, XP002424085 cited in the application

## Description

The present invention concerns substituted pyrazinone derivatives having selective α_{2C}-adrenoceptor antagonist activity. Some compounds also show moderate 5-HTT activity. It further relates to their preparation, pharmaceutical compositions comprising them and their use as a medicine, especially for the treatment of central nervous system disorders.

### Background of the Invention

Adrenergic receptors form the interface between the endogenous catecholamines epinephrine and norepinephrine and a wide array of target cells in the body to mediate the biological effects of the sympathetic nervous system. They are divided into three major subcategories, α₁, α₂ and β. To date, nine distinct adrenergic receptor subtypes have been cloned from several species: α_{1A}, α_{1B}, α_{1D}, α_{2A}, α_{2B}, α_{2C}, β₁, β₂ and β₃ (Hieble, J. P.; et al. J. Med. Chem. 1995, 38, 3415-3444). Available α₂ ligands have only marginal subtype selectivity. A complicating factor is that α₂-adrenoceptor ligands, which are imidazoles or imidazolines, also bind with moderate-to-high affinity to non-adrenoceptor imidazoline binding sites.

The three α₂-adrenoceptor subtypes share many common properties. They are G-protein-coupled receptors with seven transmembrane domains of the aminebinding subfamily. All three subtypes are coupled to the Gi/o signalling system, inhibiting the activity of adenylate cyclase, the opening of voltage-gated Ca²⁺ channels and the opening of K⁺ channels. The three receptors are encoded by distinct genes (Bylund, D. B.; et al. Pharmacol. Rev. 1994, 46, 121-136 and Hieble, J. P. et al. Pharmacol. Commun. 1995, 6, 91-97), localized to different chromosomes; in humans the gene for α_{2A} is found on chromosome 10, the α_{2B}-gene on chromosome 2 and the α_{2C}-gene on chromosome 4. The subtypes are well conserved across mammalian species. In rats and mice, however, there is a single amino acid substitution which decreases the affinity of the rodent α_{2A}-adrenoceptor for the classical α₂-antagonists, yohimbine and rauwolscine. The general consensus is that this so-called α_{2D}-adrenoceptor subtype represents the rodent homologue of the human α_{2A}-subtype.

The α₂-adrenoceptor subtypes are differentially distributed in cells and tissues, clearly endowing the receptors with different physiological functions and pharmacological activity profiles. Different regulatory regions in the receptor genes and different protein structures also confer different regulatory properties on the three receptors, both with regard to receptor synthesis and post-translational events.

α₂-Adrenergic receptors were initially characterized as presynaptic receptors that serve as parts of a negative feedback loop to regulate the release of norepinephrine. Soon it was shown that α₂-adrenoceptors are not restricted to presynaptic locations but also have postsynaptic functions. The α_{2A}-adrenoceptor is the major inhibitory presynaptic receptor (autoreceptor) regulating release of norepinephrine from sympathetic neurons as part of a feedback loop. The α_{2C}-adrenoceptor turned out to function as an additional presynaptic regulator in all central and peripheral nervous tissues investigated. However, the relative contributions of α_{2A} and α_{2C}-receptors differed between central and peripheral nerves, with the α_{2C}-subtype being more prominent in sympathetic nerve endings than in central adrenergic neurons (Philipp, M. et al. Am. J. Physiol. Regul. Integr. Comput. Physiol. 2002,283, R287-R295 and Kable, J. W. et al. J. Pharmacol. Exp. Ther. 2000, 293, 1-7). The α_{2C}-adrenoceptor is particularly suited to control neurotransmitter release at low action potential frequencies. In contrast, the α_{2A}-adrenoceptor seems to operate primarily at high stimulation frequencies in sympathetic nerves and may thus be responsible for controlling norepinephrine release during maximal sympathetic activation (Bucheler, M. M. et al. Neuroscience 2002, 109, 819-826). α_{2B}-Adrenoceptors are located on postsynaptic cells to mediate the effects of catecholamines released from sympathetic nerves, e.g., vasoconstriction. α₂-Adrenergic receptors not only inhibit release of their own neurotransmitters but can also regulate the exocytosis of a number of other neurotransmitters in the central and peripheral nervous system. In the brain, α_{2A}- and α_{2C}-adrenoceptors can inhibit dopamine release in basal ganglia as well as serotonin secretion in mouse hippocampal or brain cortex slices. In contrast, the inhibitory effect of α₂-adrenoceptor agonists on gastrointestinal motility was mediated solely by the α_{2A}-subtype. Part of the functional differences between α_{2A}- and α_{2C}-receptors may be explained by their distinct subcellular localization patterns. When expressed in rat fibroblasts, α_{2A}- and α_{2B}-adrenoceptors are targeted to the plasma membrane. On stimulation with agonist, only α_{2B}-adrenoceptors are reversibly internalized into endosomes. α_{2C}-Adrenoceptors are primarily localized in an intracellular membrane compartment, from where they can be translocated to the cell surface after exposure to cold temperature (see a.o. Docherty J.R. et. al. Eur. J. Pharmacol. 1998, 361, 1-15).

The establishment of genetically engineered mice lacking or overexpressing α₂-adrenoceptor subtypes has yielded important information for understanding the subtype specific functions (MacDonald, E. et al. Trends Pharmacol. Sci. 1997, 18, 211-219). The examination of the phenotype of these strains of mice demonstrated that the α_{2A}-subtype is responsible for inhibition of neurotransmitter release from central and peripheral sympathetic nerves and for most of the centrally mediated effects of α₂-agonists. The α_{2B} subtype is primarily responsible for the initial peripheral hypertensive responses evoked by the α₂-agonists and takes part in the hypertension induced by salt (Link et al. Science 1996, 273, 803-805 and Makaritsis, K. P. et al. Hypertension 1999, 33, 14-17).

Clarification of the physiological role of the α_{2C} subtype proved more difficult. Despite a rather wide distribution in the CNS, its role did not appear critical in the mediation of the cardiovascular effects of nonselective α₂-agonists. Its participation has been suggested in the hypothermia induced by dexmedetomidine and in the hyperlocomotion induced by D-amphetamine (Rohrer, D. K. et al. Annu. Rev. Pharmacol Toxicol. 1998, 38, 351-373). Another potentially important response mediated by the α_{2C}-adrenoceptor is constriction of cutaneous arteries, leading to a reduction in cutaneous blood flow (Chotani, M. A. et al. Am. J. Physiol. Heart Circ. Physiol. 2004, 286, 59-67). Recent studies carried out on double knockout mice have suggested that α_{2C}-adrenoceptor is also expressed at the presynaptic level where, together with α_{2A}, it actively participates in the control of neurotransmitter release. While α_{2A}-adrenoceptor is particularly efficient at high stimulation frequencies, α_{2C}-adrenoceptor acts rather at low stimulation frequencies. Moreover, it has been suggested that α_{2C} subtype participates in the modulation of motor behavior and the memory processes (Bjorklund, M. et al. Neuroscience 1999, 88, 1187-1198 and Tanila, H. et al. Eur. J. Neurosci. 1999, 11, 599-603). Other central effects triggered by this subtype include also the startle reflex and aggression response to stress and locomotion (Sallinen, J. et al. J. Neurosci. 1998, 18, 3035-3042 and Sallinen. J. et al. Neuroscience 1998, 86, 959-965). Last, it was recently pointed out that the α_{2C}-adrenoceptor might contribute to α₂-agonist-mediated spinal analgesia and adrenergic-opioid synergy (Fairbanks, C. A. et al. J. Pharm.Exp. Ther. 2002, 300, 282-290).

Because of their widespread distribution in the central nervous system, α₂-receptors affect a number of behavioral functions. The effect of altered α_{2C}-adrenergic receptor expression has been evaluated in several different behavioral paradigms (Kable J.W. et al., Journal of Pharmacology and Experimental Therapeutics, 2000, 293 (1): 1-7), proving that α_{2C}-adrenergic antagonists may have therapeutic value in the treatment of stress-related psychiatric disorders. In each of the behavioral paradigms, it is unclear whether the α_{2C}-subtype plays some direct role in mediating behavior or whether altered α_{2C}-receptor expression produces effects because of altered metabolism or downstream modulation of other neurotransmitter systems. Interestingly, α_{2C}-receptor-deficient mice had enhanced startle responses, diminished prepulse inhibition, and shortened attack latency in the isolation aggression test. Thus drugs acting via the α_{2C}-adrenoceptor may have therapeutic value in disorders associated with enhanced startle responses and sensorimotor gating deficits, such as schizophrenia, attention deficit disorder, posttraumatic stress disorder, and drug withdrawal. In addition to the α_{2C}-subtype, the α_{2A}-adrenoceptor has an important.

With more and more studies of the α₂-adrenoceptor physiology in gene-targeted mice being published, the situation becomes more complicated than initially anticipated. Indeed, only a few biological functions of α₂-receptors were found to be mediated by one single α₂-adrenergic receptor subtype. For other α₂-receptor-mediated functions, two different strategies seem to have emerged to regulate adrenergic signal transduction: some biological functions are controlled by two counteracting α₂-receptor subtypes, and some require two receptor subtypes with similar but complementary effects. Because the α_{2A}-subtype mediates most of the classical effects of α₂-adrenergic agonists, it is doubtful that an α_{2A}-selective agonist would have a substantially better clinical profile than the currently available agents. Drugs acting at α_{2B}- or α_{2C}-adrenergic receptors are likely to have fewer of the classical α₂-adrenergic side effects than α_{2A}-specific agents. It would appear likely that α_{2C}-selective agents may be useful in at least some nervous system disorders, in particular central nervous system disorders.

### Background prior art

Analysis of the pipeline databases to date indicate that there are several adrenergic α₂-antagonists in the market, by companies including Akzo Nobel (Organon), Novartis, Pfizer, and Schering AG. None of those compounds are selective for any of the three α₂-adrenoceptors. These compounds are indicated mainly for depression, hypertensive disorders and dyskinesias associated with Parkinson's disease. Companies with α₂-adrenoceptor antagonists in clinical development include Britannia Pharmaceuticals, IVAX, Juvantia Pharmaceuticals, MAP Pharmaceuticals, Novartis, Novo Nordisk, Organon, Pierre Fabre, and Sanofi-Aventis.

Regarding the development of selective α_{2C}-adrenoceptor antagonists to date, OPC-28326 is the only compound in clinical development (in Phase 2 by Otsuka Pharmaceuticals for hypertensive disorders and peripheral vascular disease). The rest of the α_{2C} antagonists are in preclinical development by Oy Juvantia Pharma Ltd (JP 1514 and JP 1302, published in WO 01/64645 and WO 04/067513) and by Novartis AG (NVP-ABE651 and NVP-ABE697, published in WO 01/55132 and J. Label Compd. Radiopharm 2002, 45, 1180), indicated mainly for depression and schizophrenia. In addition, several compounds are listed at the very early stages of development (biological testing) by Juvantia and Kyowa Hakko, for depression and Parkinson's disease.

### Description of the Invention

It is the object of the present invention to provide a compound with a binding affinity towards α₂-adrenoceptor receptors, in particular towards α_{2C}-adrenoceptor receptors, in particular as an antagonist.

This goal was achieved by a compound according to the general Formula (I) 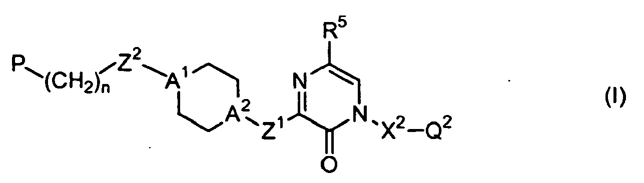 a pharmaceutically acceptable acid or base addition salt thereof, an *N*-oxide form thereof or a quaternary ammonium salt thereof, wherein :
- A¹, A²: are each, independently from each other, a nitrogen or a carbon-atom; with the provision that A¹ and A² are not simultaneously a carbon atom;
- Z¹, Z²: are each, independently from each other, a covalent bond or N-R⁴ ; wherein R⁴ is selected from the group of hydrogen, (C₁-₃)alkyl, aryl and aryl-(C₁₋₃)alkyl;
- n: is an integer equal to zero, 1, 2 or 3 ;
- R⁵: is selected from the group of hydrogen and halo ;
- P: is a radical selected from the group of phenyl, biphenyl, 1,1-diphenylmethyl and benzyloxyphenyl ;
- X²: is a covalent bond, a saturated or an unsaturated (C₁₋₈)-hydrocarbon radical, wherein one or more bivalent -CH₂-units may optionally be replaced by a respective bivalent phenyl-unit ; and/or wherein one or more hydrogen atoms may be replaced by a radical selected from the group of oxo ; (C₁₋₃)alkyloxy ; halo; cyano ; nitro ; formyl ; hydroxy ; amino ; trifluoromethyl ; mono- and di((C₁₋₃)alkyl)amino ; carboxyl ; and thio ;
- Q²: is a radical selected from the group of hydrogen ; -NR¹R² ; Pir ; -OR^{3a} ; SR^{3b} ; SO₂R^{3c} ; aryl ; and Het; wherein two radicals -OR^{3a} may be taken together to form a bivalent radical -O-(CH₂)ₛ-O- wherein s is an integer equal to 1, 2 or 3 ;
- R¹ and R²: are each, independently from each other, a radical selected from the group of hydrogen ; alkyl ; alkenyl ; alkynyl ; aryl ; arylalkyl ; diarylalkyl ; alkylcarbonyl ; alkylcarbonylalkyl ; alkenylcarbonyl ; alkyloxy ; alkyloxyalkyl ; alkyloxycarbonyl ; alkyloxyalkylcarbonyl; alkyloxycarbonylalkyl ; alkyloxycarbonylalkylcarbonyl; alkylsulfonyl ; arylsulfonyl ; arylalkylsulfonyl ; arylalkenylsulfonyl ; Hetsulfonyl ; arylcarbonyl ; aryloxyalkyl ; arylalkylcarbonyl ; Het ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein R^{a} and R^{b} are each independently selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl ;
- Pir: is a radical containing at least one N, by which it is attached to the X²-radical, selected from the group of pyrrolidinyl ; imidazolidinyl ; pyrazolidinyl ; piperidinyl ; piperazinyl ; pyrrolyl ; pyrrolinyl ; imidazolinyl ; pyrrazolinyl ; pyrrolyl ; imidazolyl ; pyrazolyl ; triazolyl ; azepyl ; diazepyl; morpholinyl ; thiomorpholinyl ; indolyl ; isoindolyl; indolinyl ; indazolyl ; benzimidazolyl ; and 1,2,3,4-tetrahydro-isoquinolinyl ; wherein each Pir-radical is optionally substituted by 1, 2 or 3 radicals selected from the group of hydroxy ; halo ; oxo ; (C₁₋₃)alkyl ; (C₁₋₃)alkenyl ; (C₁₋₃)alkyloxycarbonyl ; Het-carbonyl ; (C₁₋₃)alkylamino ; trifluoromethyl ; phenyl(₀₋₃)alkyl ; pyrimidinyl; pyrrolidinyl ; and pyridinyloxy ;
- R^{3a}: a radical selected from the group of hydrogen ; alkyl ; trihaloalkyl ; arylalkyl ; alkyloxyalkyl ; Het ; and Het-alkyl ;
- R^{3b}, R^{3c}: are each, independently from each other, a radical selected from the group of hydrogen ; alkyl ; trihaloalkyl ; aryl ; arylalkyl ; alkyloxyalkyl ; Het; and Het-alkyl ;
- Het: is a heterocyclic radical selected from the group of pyrrolidinyl ; imidazolidinyl ; pyrazolidinyl ; piperidinyl ; piperazinyl ; pyrrolyl ; pyrrolinyl ; imidazolinyl ; pyrrazolinyl ; pyrrolyl ; imidazolyl ; pyrazolyl ; triazolyl ; pyridinyl ; pyridazinyl ; pyrimidinyl ; pyrazinyl ; triazinyl ; azepyl ; diazepyl ; morpholinyl ; thiomorpholinyl ; indolyl ; isoindolyl ; indolinyl ; indazolyl ; benzimidazolyl ; 1,2,3,4-tetrahydro-isoquinolinyl ; furyl ; tetrahydropyranyl ; thienyl ; oxazolyl ; isoxazolyl ; thiazolyl ; thiadiazolyl ; isothiazolyl ; dioxolyl ; dithianyl ; tetrahydrofuryl ; tetrahydropyranyl ; oxadiazolyl ; quinolinyl ; isoquinolinyl ; quinoxalinyl ; benzoxazolyl ; benzisoxazolyl; benzothiazolyl; benzisothiazolyl ; benzofuranyl ; benzothienyl ; benzopiperidinyl ; benzomorpholinyl ; chromenyl ; and imidazo[1,2-a]pyridinyl ; wherein each Het-radical is optionally substituted by one or more radicals selected from the group of halo ; oxo ; (C₁₋₃)alkyl ; phenyl, optionally substituted with (C₁₋₃)alkyloxy ; (C₁₋₃)alkylcarbonyl ; (C₁₋₃)alkenylthio ; imidazolyl-(C₁₋₃)alkyl ; aryl(C₁₋₃)-alkyl and (C₁₋₃)alkyloxycarbonyl ;
- aryl: is naphthyl or phenyl, each optionally substituted with 1, 2 or 3 substituents, each independently from each other, selected from the group of oxo ; (C₁₋₃)alkyl; (C₁₋₃)alkyloxy ; halo ; cyano ; nitro ; formyl ; ethanoyl ; hydroxy; amino ; trifluoromethyl ; mono- and di((C₁₋₃)alkyl)amino ; mono- and di((C₁₋₃)alkylcarbonyl)amino ; carboxyl; morpholinyl ; and thio ;
- alkyl: is, unless otherwise indicated, a straight or branched saturated hydrocarbon radical having from 1 to 8 carbon atoms; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms; wherein each radical is optionally substituted on one or more carbon atoms with one or more radicals selected from the group of oxo ; (C₁₋₃)alkyloxy, halo ; cyano ; nitro ; formyl ; hydroxy; amino ; carboxyl ; and thio ;
- alkenyl: is an alkyl radical as defined above, further having one or more double bonds;
- alkynyl: is an alkyl radical as defined above, further having one or more triple bonds;
- arylalkyl: is an alkyl radical as defined above, further having one CH₃-group replaced by phenyl ; and
- diarylalkyl: is an alkyl radical as defined above, further having two CH₃-groups replaced by phenyl.

The invention also relates to a pharmaceutical composition comprising a pharmaceutically acceptable carrier or diluent and, as active ingredient, a therapeutically effective amount of a compound according to the invention, in particular a compound according to Formula (I), a pharmaceutically acceptable acid or base addition salt thereof, an *N*-oxide form thereof or a quaternary ammonium salt thereof.

The invention also relates to the use of a compound according to the invention for the preparation of a medicament for the prevention and/or treatment of a disorder or disease responsive to antagonism of the α₂-adrenergic receptor, in particular to antagonism of the α_{2C}-adrenergic receptor.

In particular, the invention relates to the use of a compound according to the invention for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders and sexual dysfunction.

A compound according to the invention may also be suitable as add-on treatment and/or prophylaxis in the above listed diseases in combination with antidepressants, anxiolytics and/or antipsychotics which are currently available or in development or which will become available in the future, to improve efficacy and/or onset of action. This is evaluated in rodent models in which antidepressants, anxiolytics and/or antipsychotics are shown to be active. For example, compounds are evaluated in combination with antidepressants, anxiolytics and/or antipsychotics for attenuation of stress-induced hyperthermia.

The invention therefore also relates to the use of a compound according to the invention for use as an add-on treatment with one or more other compounds selected from the group of antidepressants, anxiolytics and antipsychotics, to a pharmaceutical composition comprising a compound according to the invention and one or more other compounds selected from the group of antidepressants, anxiolytics and antipsychotics, as well as to a process for the preparation of such pharmaceutical compositions and to the use of such a composition for the manufacture of a medicament, in particular to improve efficacy and/or onset of action in the treatment of depression and/or anxiety.

### Detailed description of the invention

In a preferred embodiment, the invention relates to a compound according to the invention, wherein the moiety 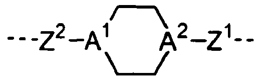 is a bivalent radical of formula (II-a), (II-b), (II-c) and (II-d), as shown below.

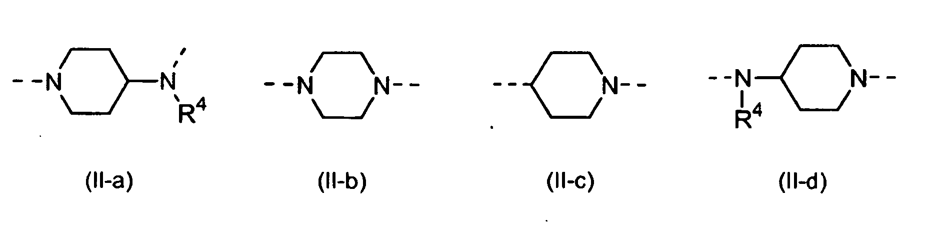

In a preferred embodiment, the invention relates to a compound according to the invention, wherein R⁴ is hydrogen or p-aminomethylbenzyl. Most preferably, R⁴ is hydrogen.

In a preferred embodiment, the invention relates to a compound according to the invention, wherein n is 1, 2 or 3.

In a preferred embodiment, the invention relates to a compound according to the invention, wherein R⁵ is hydrogen.

In a preferred embodiment, the invention relates to a compound according to the invention, wherein P is phenyl.

In a preferred embodiment, the invention relates to a compound according to the invention, wherein X² is selected from the group of a covalent bond, a C₁-hydrocarbon radical, a C₂-hydrocarbon radical, or a C₃-hydrocarbon radical.

In one preferred embodiment of X², one bivalent -CH₂-unit of the hydrocarbon radical X² is replaced by a bivalent phenyl-unit ; or wherein two hydrogen atoms of the hydrocarbon radical X² are replaced by an oxo-radical.

In a further preferred embodiment, the invention relates to a compound according to the invention, wherein X² is selected from the group of a covalent bond and any one of the radicals (aa) to (bm) defined as :

| | | | |
|---|---|---|---|
| -CH₂- | (aa) | 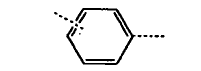 | (ba) |
| -CH₂CH₂- | (ab) | 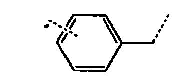 | (bb) |
| -CH₂CH₂CH₂- | (ac) | 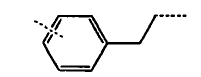 | (bc) |
| -CH₂CH₂CH₂CH₂- | (ad) | 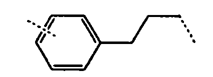 | (bd) |
| -CH₂CH₂CH₂CH₂CH₂ | (ae) | 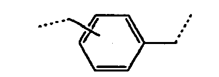 | (be) |
| -CH₂CH(CH-)₂ | (af) | 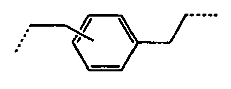 | (bf) |
| -CH₂CH=CH- | (ag) | 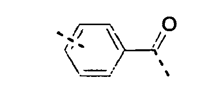 | (bg) |
| -CH₂CH=CHCH₂- | (ah) | 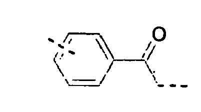 | (bh) |
| -CH₂C≡CCH₂- | (ai) | 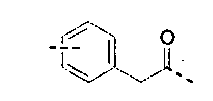 | (bi) |
| -CH(CH₃)CH(CH₃)- | (aj) | 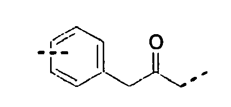 | (bj) |
| 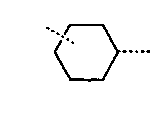 | (ak) | 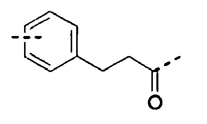 | (bk) |
| 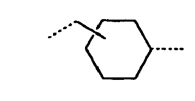 | (al) | 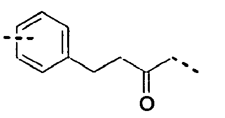 | (bl) |
| -C(=O)CH₂- | (am) | 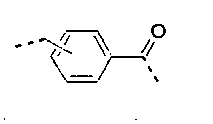 | (bm) |
| -C(=O)CH₂CH₂- | (an) | | |
| -C(=O)CH₂CH₂CH₂- | (ao) | | |
| -C(=O)CH₂CH₂CH₂CH₂- | (ap) | | |
| -CH₂C(=O)CH₂- | (aq) | | |
| -CH₂C(=O) CH₂CH₂- | (ar) | | |
| -CH₂C(=O)C(CH₃)₂CH₂- | (as) | | |

It is within the ambit of the invention that each of the radicals can be used as a linker in which either the left side (left bond) of the linker or the right side (right bond) of the linker is connected to the central pyrazinone-moiety. This is particulary relevant for non-symmetrical linkers that can thus be used in two configurations.

In a further preferred embodiment, the invention relates to a compound according to the invention, wherein X² is selected from the group of a covalent bond and any one of the radicals (aa), (ab), (ac), (ag), (am), (an), (aq), (as) and (be) as defined below :

| | | | |
|---|---|---|---|
| -CH₂- | (aa) | 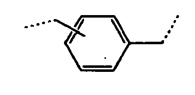 | (be) |
| -CH₂CH₂- | (ab) | | |
| -CH₂CH₂CH₂- | (ac) | | |
| -CH₂CH=CH- | (ag) | | |
| -C(=O)CH₂- | (am) | | |
| -C(=O)CH₂CH₂- | (an) | | |
| -CH₂C(=O)CH₂- | (aq) | | |
| -CH₂C(=O)C(CH₃)₂CH₂- | (as) | | |

In every embodiment of this invention, when X² is or contains a cyclic unit, i.e. a phenyl unit or a cyclohexyl unit, the attachments to the unit can be in ortho, meta or para-position ; preferably the attachments to the unit are in meta or para-position, most preferably in para-position.

In a preferred embodiment, the invention relates to a compound wherein Q² is a radical selected from the group of hydrogen ; -NR¹R² ; Pir ; -OR^{3a} ; SR^{3b} ; aryl ; and Het.

In a preferred embodiment, the invention relates to a compound wherein R¹ and R² are each, independently from each other, a radical selected from the group of hydrogen ; alkyl ; and alkyloxycarbonyl.

Preferably, when R¹ or R² comprises an alkyl moiety, the alkyl moiety is methyl ; *t*-butyl ; or a bivalent moiety derived therefrom in the sense that one hydrogen is replaced by a bond to form a bivalent radical, such as for instance is the case for alkyl in the moiety phenylalkyl.

In a further preferred embodiment, the invention relates to a compound according to the invention, wherein Pir is a radical containing at least one N, by which it is attached to the X²-radical, selected from the group of piperidinyl and isoindolyl ; wherein each Pir-radical is optionally substituted by 2 oxo-radicals, such as, for instance, isoindolyl-1,3-dione.

In a further preferred embodiment, the invention relates to a compound according to the invention, wherein R^{3a} and R^{3b} are each, independently from each other, an alkyl-radical. Preferably, R^{3a} and R^{3b} are each, independently from each other, methyl.

In a further embodiment, the invention relates to a compound according to the invention, wherein Het is a heterocyclic radical selected from the group of pyridinyl ; furyl ; tetrahydropyranyl; thienyl ; oxadiazolyl ; and quinolinyl ; wherein each Het-radical is optionally substituted by one or more radicals selected from the group of halo and phenyl, optionally substituted with (C₁₋₃)alkyloxy.

Most preferably, the invention relates to a compound according to the invention, wherein aryl is naphthyl or phenyl, each optionally substituted with a substituent, selected from the group of (C₁₋₃)alkyl and halo.

In a further preferred embodiment, the invention relates to a compound according to the invention, wherein
- A¹, A²: are each, independently from each other, a nitrogen or a carbon-atom; with the provision that A¹ and A² are not simultaneously a carbon atom;
- Z¹, Z²: are each, independently from each other, a covalent bond or N-R⁴ ; wherein R⁴ is selected from the group of hydrogen and aryl-(C₁₋₃)alkyl ;
- n: is an integer equal to zero, 1, 2 or 3 ;
- R⁵: is hydrogen ;
- P: is a radical selected from the group of phenyl, biphenyl, 1,1-diphenylmethyl and benzyloxyphenyl ;
- X²: is a bond, a saturated or an unsaturated (C₁₋₈)-hydrocarbon radical, wherein one or more bivalent -CH₂-units may optionally be replaced by a respective bivalent phenyl-unit ; and/or wherein one or more hydrogen atoms may be replaced by an oxo-radical ;
- Q²: is a radical selected from the group of hydrogen ; -NR¹R² ; Pir ; -OR^{3a} ; SR^{3b} ; aryl ; and Het;
- R¹ and R²: are each, independently from each other, a radical selected from the group of hydrogen ; alkyl ; and alkyloxycarbonyl ;
- Pir: is a radical containing at least one N, by which it is attached to the X²-radical, selected from the group of piperidinyl ; isoindolyl; wherein each Pir-radical is optionally substituted by 2 oxo-radicals ;
- R^{3a}, R^{3b}, R^{3c}: are each, independently from each other, an alkyl-radical ;
- Het: is a heterocyclic radical selected from the group of pyridinyl ; furyl ; tetrahydropyranyl ; thienyl ; oxadiazolyl ; and quinolinyl ; wherein each Het-radical is optionally substituted by one or more radicals selected from the group of halo ; and phenyl, optionally substituted with (C₁₋₃)alkyloxy ; and
- aryl: is naphthyl or phenyl, each optionally substituted with a substituent, each independently from each other, selected from the group of (C₁₋₃)alkyl and halo.

In the framework of this application, and unless the number of carbon atoms is indicated differently, alkyl is a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms being part of a straight or branched saturated hydrocarbon radical having from 1 to 8 carbon atoms; wherein each radical is optionally substituted on one or more carbon atoms with one or more radicals selected from the group of oxo ; (C₁₋₃)alkyloxy ; halo ; cyano ; nitro; formyl ; hydroxy ; amino ; carboxy ; and thio. Preferably, alkyl is methyl, ethyl, propyl, isopropyl, butyl, *tert*-butyl, pentyl, hexyl, pentyl, octyl, cyclopropyl, cyclopentyl, cyclohexyl, cyclohexylmethyl and cyclohexylethyl. Most preferably, alkyl is a (C₁₋₂)alkyl radical.

In the framework of this application, alkenyl is an alkyl radical as defined above having one or more double bonds. Preferably, alkenyl is ethenyl, propenyl and butynyl.

In the framework of this application, alkynyl is an alkyl radical as defined above having one or more triple bonds. Preferably, alkynyl is ethynyl and propynyl.

In the framework of this application, arylalkyl is an alkyl radical as defined above, having one CH₃-radical replaced by a phenyl-radical. An examples of such a radical is benzyl.

In the framework of this application, diarylalkyl is an alkyl radical as defined above, having two CH₃-radical replaced by a phenyl-radical. An examples of such a radical is diphenylmethyl and 1,1-diphenylethyl.

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 7 carbon atoms, wherein one or more carbon atoms is substituted with one or more halo atoms. Preferably, halo is bromo, fluoro or chloro ; more preferably, halo is fluoro. Preferably, haloalkyl is trifluoroalkyl ; more preferably haloalkyl is trifluoromethyl.

In the framework of this application, unless otherwise indicated, a bond can be any bond, including a covalent bond, a single bond, a double bond, a triple bond, a coordination bond and a hydrogen bond.

In the framework of this application, with "a compound according to the invention" is meant a compound according to the general Formula (I), a pharmaceutically acceptable acid or base addition salt thereof, an *N*-oxide form thereof, or a quaternary ammonium salt thereof.

A pharmaceutically acceptable acid addition salt is defined to comprise a therapeutically active non-toxic acid addition salt form that a compound according to Formula (I) is able to form. Said salt can be obtained by treating the base form of a compound according to Formula (I) with an appropriate acid, for example an inorganic acid, for example hydrohalic acid, in particular hydrochloric acid, hydrobromic acid, sulphuric acid, nitric acid and phosphoric acid ; an organic acid, for example acetic acid, hydroxyacetic acid, propanoic acid, lactic acid, pyruvic acid, oxalic acid, malonic acid, succinic acid; maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid.

Conversely said acid addition salt form may be converted into the free base form by treatment with an appropriate base .

The compound according to Formula (I) containing an acidic proton may also be converted into a therapeutically active non-toxic metal or amine addition salt form (base addition salt) by treatment with an appropriate organic and inorganic base. Appropriate base salt forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.

Conversely, said salt form can be converted into the free form by treatment with an appropriate acid.

The term addition salt as used in the framework of this application also comprises a solvate that the compound according to Formula (I), as well as a salt thereof, is able to form. Such solvates are, for example, hydrates and alcoholates.

The *N*-oxide form of the compound according to Formula (I) is meant to comprise a compound of Formula (I) wherein one or several nitrogen atoms are oxidized to so-called *N*-oxides, particularly those *N*-oxides wherein one or more tertiary nitrogens (e.g. of the piperazinyl or piperidinyl radical) are *N*-oxidized. Such *N*-oxides can easily be obtained by a skilled person without any inventive skills and they are obvious alternatives for a compound according to Formula (I) since these compounds are metabolites, which are formed by oxidation in the human body upon uptake . As is generally known, oxidation is normally the first step involved in drug metabolism (Textbook of Organic Medicinal and Pharmaceutical Chemistry, 1977, pages 70- 75). As is also generally known, the metabolite form of a compound can also be administered to a human instead of the compound per se, with much the same effects.

A compound of Formula (I) may be converted to the corresponding *N*-oxide form following art-known procedures for converting a trivalent nitrogen into its *N*-oxide form. Said *N*-oxidation reaction may generally be carried out by reacting the compound of Formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert*-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

A quaternary ammonium salt of compound according to Formula (I) defines said compound which is able to form by a reaction between a basic nitrogen of a compound according to Formula (I) and an appropriate quaternizing agent, such as, for example, an optionally substituted alkylhalide, arylhalide or arylalkylhalide, in particular methyliodide and benzyliodide. Other reactants with good leaving groups may also be used, such as, for example, alkyl trifluoromethanesulfonates, alkyl methanesulfonates and alkyl p-toluenesulfonates. A quaternary ammonium salt has at least one positively charged nitrogen. Pharmaceutically acceptable counterions include chloro, bromo, iodo, trifluoroacetate and acetate ions.

The invention also comprises a derivative compound (usually called "pro-drug") of a pharmacologically-active compound according to the invention, in particular according to Formula (I), which is degraded *in vivo* to yield a compound according to the invention. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

A pro-drug form of a pharmacologically-active compound according to the invention will generally be a compound according to Formula (I), a pharmaceutically acceptable acid or base addition salt thereof, an N-oxide form thereof, or a quaternary ammonium salt thereof, having an acid group which is esterified or amidated. Included in such esterified acid groups are groups of the formula -COOR^{x}, where R^{x} is a C₁₋₆alkyl, phenyl, benzyl or one of the following groups :

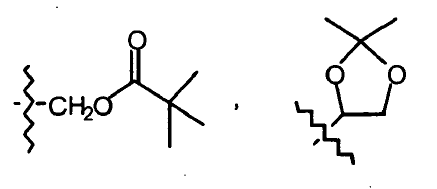

Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl. A compound according to the invention having an amino group may be derivatised with a ketone or an aldehyde such as formaldehyde to form a Mannich base. This base will hydrolyze with first order kinetics in aqueous solution.

In the framework of this application, a compound according to the invention is inherently intended to comprise all stereochemically isomeric forms thereof. The term "stereochemically isomeric form" as used herein defines all the possible isomeric forms that a compound of Formula (I) may possess. Unless otherwise mentioned or indicated, the chemical designation of a compound denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Compounds encompassing double bonds can have an E or Z-stereochemistry at said double bond. Hence, all stereochemically isomeric forms of a compound of Formula (I) are intended to be embraced within the scope of this invention.

Following CAS nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R*,R* ]* or [*R*,S*],* where R* is always specified as the reference center and [*R*,R**] indicates centers with the same chirality and [*R*,S**] indicates centers of unlike chirality. For example, if the lowest-numbered chiral center in the molecule has an S configuration and the second center is *R*, the stereo descriptor would be specified as *S*-[*R***,S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system (hydrogen atom in a compound according to Formula (I)) relative to the position of the highest priority substituent on the reference atom is denominated "α", if it is on the same side of the mean plane determined by the ring system, or "β", if it is on the other side of the mean plane determined by the ring system.

In the framework of this application, a compound according to the invention is inherently intended to comprise all isotopic combinations of its chemical elements. In the framework of this application, a chemical element, in particular when mentioned in relation to a compound according to Formula (I), comprises all isotopes and isotopic mixtures of this element, either naturally occuring or synthetically produced, either with natural abundance or in an isotopically enriched form. In particular, when hydrogen is mentioned, it is understood to refer to ¹H, ²H, ³H and mixtures thereof ; when carbon is mentioned, it is understood to refer to ¹¹C, ¹²C, ¹³C, ¹⁴C and mixtures thereof ; when nitrogen is mentioned, it is understood to refer to ¹³N, ¹⁴N, ¹⁵N and mixtures thereof ; when oxygen is mentioned, it is understood to refer to ¹⁴O, ¹⁵O, ¹⁶O, ¹⁷O, ¹⁸O and mixtures thereof ; and when fluor is mentioned, it is understood to refer to ¹⁸F, ¹⁹F and mixtures thereof.

A compound according to the invention therefore inherently comprises a compound with one or more isotopes of one or more element, and mixtures thereof, including a radioactive compound, also called radiolabelled compound, wherein one or more non-radioactive atoms has been replaced by one of its radioactive isotopes. By the term "radiolabelled compound" is meant any compound according to Formula (I), a pharmaceutically acceptable acid or base addition salt thereof, an N-oxide form thereof, or a quaternary ammonium salt thereof, which contains at least one radioactive atom. For example, a compound can be labelled with positron or with gamma emitting radioactive isotopes. For radioligand-binding techniques (membrane receptor assay), the ³H-atom or the ¹²⁵I-atom is the atom of choice to be replaced. For imaging, the most commonly used positron emitting (PET) radioactive isotopes are ¹¹C, ¹⁸F, ¹⁵O and ¹³N, all of which are accelerator produced and have half-lives of 20, 100, 2 and 10 minutes respectively. Since the half-lives of these radioactive isotopes are so short, it is only feasible to use them at institutions which have an accelerator on site for their production, thus limiting their use. The most widely used of these are ¹⁸F, ^{99m}Tc, ²⁰¹Tl and ¹²³I. The handling of these radioactive isotopes, their production, isolation and incorporation in a molecule are known to the skilled person.

In particular, the radioactive atom is selected from the group of hydrogen, carbon, nitrogen, sulfur, oxygen and halogen. Preferably, the radioactive atom is selected from the group of hydrogen, carbon and halogen.

In particular, the radioactive isotope is selected from the group of ³H, ¹¹C, ¹⁸F, ¹²²I, ¹²³I, ¹²⁵I, ¹³¹I, ⁷⁵Br, ⁷⁶Sr, ⁷⁷Br and ⁸²Br. Preferably, the radioactive isotope is selected from the group of ³H, ¹¹C and ¹⁸F.

### Preparation

A compound according to the invention can generally be prepared by a succession of steps, each of which is known to the skilled person. In particular, a pyrazinone derivative can be prepared according to one or more of the following preparation methods.

### Preparation of the final compound (I-a).

Final compound I-a, which was also used as intermediate for other final compounds, can generally be prepared as shown in Scheme 1A. 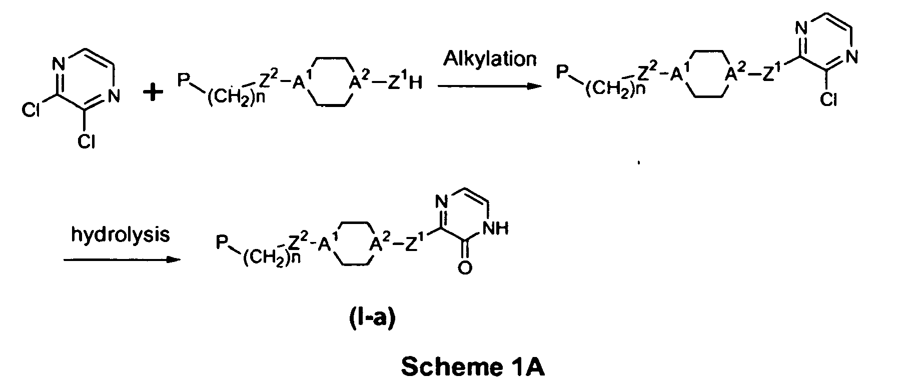 Alkylation reactions of the starting material 2,3-dichloropyrazine with aminoderivatives (Scheme 1A) may be performed in an aprotic solvent, such as, for instance DMF or DMSO, in the presence of an inorganic base, such as K₂CO₃, Na₂CO₃, NaOH or KOH, at a convenient temperature, either by conventional heating or under microwave irradiation, for a period of time to ensure the completion of the reaction, which may typically be about 16 hours under conventional heating.
Hydrolysis reactions may be performed either in acidic inorganic solvents, such as 10% HCl_{aq}, using a co-solvent such as THF, by conventional heating or under microwave heating, for a period of time to ensure the completion of the reaction, which may typically be about 16 hours under conventional heating, or under basic conditions, such as in NaOH_{aq} or in a DMSO solvent, for a period of time to ensure the completion of the reaction, which may typically be about 0.5 hours under microwave irradiation.
The final compound (I-a) is the starting compound for the compounds of the reaction schemes below. Variables Z¹, Z², A¹, A², n, P are defined as in Formula (I), unless otherwise specified.
Compound I-a can also be prepared starting from intermediate I-b by reaction with a suitable aldehyde, according to Scheme 1B. 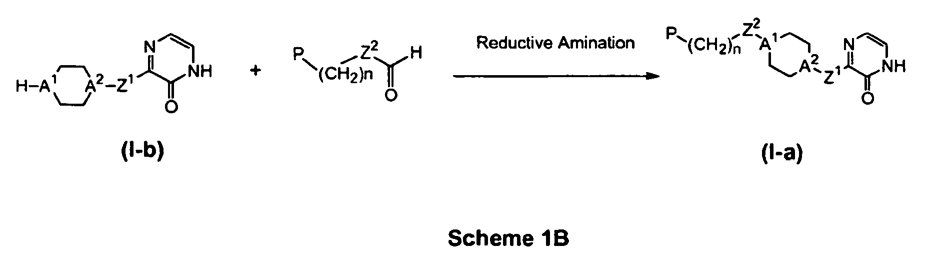 The reductive amination reaction may be performed in an aprotic solvent such as 1,2-dichloroethane, in the presence of the reducing agent such as sodium triacetoxyborohydride, for a period of time to ensure the completion of the reaction, which may typically be about 16 hours at room temperature.

### Preparation of a final compound in which X² is a saturated or an unsaturated hydrocarbon radical

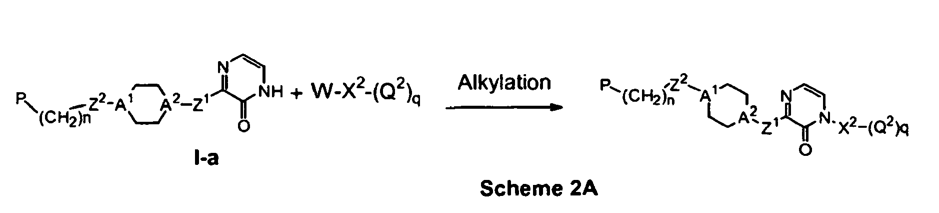

The W-radical in the compound W-X²-(Q²)_{q} is a leaving group, such as for instance Cl-, Br-, MeSO₂O- and p-MePhSO₂O- ; X² is a (C₁₋₈)-hydrocarbon radical, more preferably a (C₁₋₆)-hydrocarbon radical, even more preferably a (C₁₋₅)-hydrocarbon radical, most preferably a (C₁₋₄)-hydrocarbon radical. Variables Z¹, Z², A¹, A², n, P are defined as in Formula (I), . The alkylation reaction may be performed in an aprotic solvent, such as CH₃CN, DMF or THF in the presence of an inorganic base, such as K₂CO₃, Na₂CO₃, Cs₂CO₃, or an organic base such as TBD, PS-TBD, at a convenient temperature, either under conventional heating or microwave irradiation, for a period of time to ensure the completion of the reaction, which may typically be about 20 minutes at about 120° C under microwave irradiation.

### Preparation of a final compound : primary amines

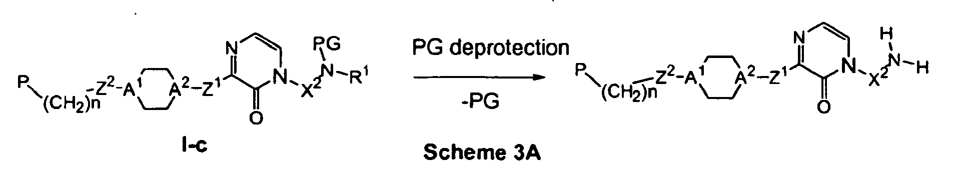

In the case where fragment -(Q²)_{q} is equal to NH₂, first the corresponding compound I-c, in which the amino group is protected with a protecting group, can be synthesized according to scheme 2A. Then, deprotection reaction may be carried out by synthetic methods well known to the person skilled in the art according to scheme 3A. Variables Z¹, Z², A¹, A², n, P are defined as in Formula (I).

### Pharmacology

A compound according to the invention, in particular compound according to Formula (I), a pharmaceutically acceptable acid or base addition salt thereof, an *N-*oxide form thereof, or a quaternary ammonium salt thereof, has surprisingly been shown to have a binding affinity towards α₂-adrenergic receptor, in particular towards α_{2C}-adrenergic receptor, in particular as an antagonist.

In view of their above mentioned potency, a compound according to the invention is suitable for the prevention and/or treatment of diseases where antagonism of the α₂-adrenergic receptor, in particular antagonism of the α_{2C}-adrenergic receptor is of therapeutic use. In particular, a compound according to the invention may be suitable for treatment and/or prophylaxis in the following diseases :
- Central nervous system disorders, including :

- Mood disorders, including particularly major depressive disorder, depression with or without psychotic features, catatonic features, melancholic features, atypical features of postpartum onset and, in the case of recurrent episodes, with or without seasonal pattern, dysthymic disorder, bipolar I disorder, bipolar II disorder, cyclothymic disorder, recurrent brief depressive disorder, mixed affective disorder, bipolar disorder not otherwise specified, mood disorder due to a general medical condition, substance-induced mood disorder, mood disorder not otherwise specified, seasonal affective disorder and premenstrual dysphoric disorders.
- Anxiety disorders, including panic attack, agoraphobia, panic disorder without agoraphobia, agoraphobia without history of panic disorder, specific phobia, social phobia, obsessive-compulsive disorder, posttraumatic stress disorder, acute stress disorder, generalized anxiety disorder, anxiety disorder due to a general medical condition, substance-induced anxiety disorder and anxiety disorder not otherwise specified.
- Stress-related disorders associated with depression and/or anxiety, including acute stress reaction, adjustment disorders (brief depressive reaction, prolonged depressive reaction, mixed anxiety and depressive reaction, adjustment disorder with predominant disturbance of other emotions, adjustment disorder with predominant disturbance of conduct, adjustment disorder with mixed disturbance of emotions and conduct, adjustment disorders with other specified predominant symptoms) and other reactions to severe stress.
- Dementia, amnesic disorders and cognitive disorders not otherwise specified, especially dementia caused by degenerative disorders, lesions, trauma, infections, vascular disorders, toxins, anoxia, vitamin deficiency or endocrinic disorders, or amnesic disorders caused by alcohol or other causes of thiamine deficiency, bilateral temporal lobe damage due to Herpes simplex encephalitis and other limbic encephalitis, neuronal loss secondary to anoxia / hypoglycaemia / severe convulsions and surgery, degenerative disorders, vascular disorders or pathology around ventricle III.
- Cognitive disorders, in particular due to cognitive impairment resulting from other medical conditions.
- Personality disorders, including paranoid personality disorder, schizoid personality disorder, schizotypical personality disorder, antisocial personality disorder, borderline personality disorder, histrionic personality disorder, narcissistic personality disorder, avoidant personality disorder, dependent personality disorder, obsessive-compulsive personality disorder and personality disorder not otherwise specified.
- Schizoaffective disorders resulting from various causes, including schizoaffective disorders of the manic type, of the depressive type, of mixed type, paranoid, disorganized, catatonic, undifferentiated and residual schizophrenia, schizophreniform disorder, schizoaffective disorder, delusional disorder, brief psychotic disorder, shared psychotic disorder, substance-induced psychotic disorder and psychotic disorder not otherwise specified.
- Akinesia, akinetic-rigid syndromes, dyskinesia and medication-induced parkinsonism, Gilles de la Tourette syndrome and its symptoms, tremor, chorea, myoclonus, tics and dystonia.
- Attention-deficit / hyperactivity disorder (ADHD).
- Parkinson's disease, drug-induced Parkinsonism, post-encephalitic Parkinsonism, progressive supranuclear palsy, multiple system atrophy, corticobasal degeneration, parkinsonism-ALS dementia complex and basal ganglia calcification.
- Dementia of the Alzheimer's type, with early or late onset, with depressed mood.
- Behavioural disturbances and conduct disorders in dementia and the mentally retarded, including restlessness and agitation.
- Extra-pyramidal movement disorders.
- Down's syndrome.
- Akathisia.
- Eating Disorders, including anorexia nervosa, atypical anorexia nervosa, bulimia nervosa, atypical bulimia nervosa, overeating associated with other psychological disturbances, vomiting associated with other psychological disturbances and non-specified eating disorders.
- AIDS-associated dementia.
- Chronic pain conditions, including neuropathic pain, inflammatory pain, cancer pain and post-operative pain following surgery, including dental surgery. These indications might also include acute pain, skeletal muscle pain, low back pain, upper extremity pain, fibromyalgia and myofascial pain syndromes, orofascial pain, abdominal pain, phantom pain, tic douloureux and atypical face pain, nerve root damage and arachnoiditis, geriatric pain, central pain and inflammatory pain.
- Neurodegenerative diseases, including Alzheimer's disease, Huntington's chorea, Creutzfeld-Jacob disease, Pick's disease, demyelinating disorders, such as multiple sclerosis and ALS, other neuropathies and neuralgia, multiple sclerosis, amyotropical lateral sclerosis, stroke and head trauma.
- Addiction disorders, including :

- Substance dependence or abuse with or without physiological dependence, particularly where the substance is alcohol, amphetamines, amphetamine-like substances, caffeine, cannabis, cocaine, hallucinogens, inhalants, nicotine, opioids, phencyclidine, phencyclidine-like compounds, sedative-hypnotics, benzodiazepines and/or other substances, particularly useful for treating withdrawal from the above substances and alcohol withdrawal delirium.
- Mood disorders induced particularly by alcohol, amphetamines, caffeine, cannabis, cocaine, hallucinogens, inhalants, nicotine, opioids, phencyclidine, sedatives, hypnotics, anxiolitics and other substances.
- Anxiety disorders induced particularly by alcohol, amphetamines, caffeine, cannabis, cocaine, hallucinogens, inhalants, nicotine, opioids, phencyclidine, sedatives, hypnotics, anxiolitics and other substances and adjustment disorders with anxiety.
- Smoking cessation.
- Body weight control, including obesity.
- Sleep disorders and disturbances, including :

- Dyssomnias and/or parasomnias as primary sleep disorders, sleep disorders related to another mental disorder, sleep disorder due to a general medical condition and substance-induced sleep disorder.
- Circadian rhythms disorders.
- Improving the quality of sleep.
- Sexual dysfunction, including sexual desire disorders, sexual arousal disorders, orgasmic disorders, sexual pain disorders, sexual dysfunction due to a general medical condition, substance-induced sexual dysfunction and sexual dysfunction not otherwise specified.

The invention therefore relates to a compound according to the invention for use as a medicine.

The invention also relates to the use of a compound according to the invention for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders and sexual dysfunction.

A compound according to the invention may be co-administered as add-on treatment and/or prophylaxis in the above listed diseases in combination with antidepressants, anxiolytics and/or antipsychotics which are currently available or in development or which will become available in the future, in particular to improve efficacy and/or onset of action. It will be appreciated that a compound of the present invention and the other agents may be present as a combined preparation for simultaneous, separate or sequential use for the prevention and/or treatment of depression and/or anxiety. Such combined preparations may be, for example, in the form of a twin pack. It will also be appreciated that a compound of the present invention and the other agents may be administered as separate pharmaceutical compositions, either simultaneously or sequentially.

The invention therefore relates to the use of a compound according to the invention as an add-on treatment in combination with one or more other compounds selected from the group of antidepressants, anxiolytics and antipsychotics.

Suitable classes of antidepressant agents include norepinephrine reuptake inhibitors, selective serotonin reuptake inhibitors (SSRI's), monoamine oxidase inhibitors (MAOI's), reversible inhibitors of monoamine oxidase (RIMA's), serotonin and noradrenaline reuptake inhibitors (SNRI's), noradrenergic and specific serotonergic antidepressants (NaSSA's), corticotropin releasing factor (CRF) antagonists, α-adrenoreceptor antagonists and atypical antidepressants.

Suitable examples of norepinephrine reuptake inhibitors include amitriptyline, clomipramine, doxepin, imipramine, trimipramine, amoxapine, desipramine, maprotiline, nortriptyline, protriptyline, reboxetine and pharmaceutically acceptable salts thereof.

Suitable examples of selective serotonin reuptake inhibitors include fluoxetine, fluvoxamine, paroxetine, sertraline and pharmaceutically acceptable salts thereof. Suitable examples of monoamine oxidase inhibitors include isocarboxazid, phenelzine, tranylcypromine, selegiline and pharmaceutically acceptable salts thereof.

Suitable examples of reversible inhibitors of monoamine oxidase include moclobemide and pharmaceutically acceptable salts thereof.

Suitable examples of serotonin and noradrenaline reuptake inhibitors include venlafaxine and pharmaceutically acceptable salts thereof.

Suitable atypical antidepressants include bupropion, lithium, nefazodone, trazodone, viloxazine, sibutramine and pharmaceutically acceptable salts thereof.

Other suitable antidepressants include adinazolam, alaproclate, amineptine, amitriptyline/chlordiazepoxide combination, atipamezole, azamianserin, bazinaprine, befuraline, bifemelane, binodaline, bipenamol, brofaromine, bupropion, caroxazone, cericlamine, cianopramine, cimoxatone, citalopram, clemeprol, clovoxamine, dazepinil, deanol, demexiptiline, dibenzepin, dothiepin, droxidopa, enefexine, estazolam, etoperidone, femoxetine, fengabine, fezolamine, fluotracen, idazoxan, indalpine, indeloxazine, iprindole, levoprotiline, litoxetine, lofepramine, medifoxamine, metapramine, metralindole, mianserin, milnacipran, minaprine, mirtazapine, monirelin, nebracetam, nefopam, nialamide, nomifensine, norfluoxetine, orotirelin, oxaflozane, pinazepam, pirlindone, pizotyline, ritanserin, rolipram, sercloremine, setiptiline, sibutramine, sulbutiamine, sulpiride, teniloxazine, thozalinone, thymoliberin, tianeptine, tiflucarbine, tofenacin, tofisopam, toloxatone, tomoxetine, veralipride, viqualine, zimelidine and zometapine and pharmaceutically acceptable salts thereof, and St. John's wort herb, or *Hypericum perforatum*, or extracts thereof.

Suitable classes of anti-anxiety agents include benzodiazepines and 5-HT_{1A} receptor agonists or antagonists, especially 5-HT_{1A} partial agonists, corticotropin releasing factor (CRF) antagonists, compounds having muscarinic cholinergic activity and compounds acting on ion channels. In addition to benzodiazepines, other suitable classes of anti-anxiety agents are nonbenzodiazepine sedative-hypnotic drugxs such as zolpidem; mood-stabilizing drugs such as clobazam, gabapentin, lamotrigine, loreclezole, oxcarbamazepine, stiripentol and vigabatrin; and barbiturates.

Suitable antipsychotic agents are selected from the group consisting of acetophenazine, in particular the maleate salt; alentemol, in particular the hydrobromide salt; alpertine; azaperone; batelapine, in particular the maleate salt; benperidol; benzindopyrine, in particular the hydrochloride salt; brofoxine; bromperidol; butaclamol, in particular the hydrochloride salt; butaperazine; carphenazine, in particular the maleate salt; carvotroline, in particular the hydrochloride salt; chlorpromazine; chlorprothixene; cinperene; cintriamide; clomacran, in particular the phosphate salt; clopenthixol; clopimozide; clopipazan, in particular the mesylate salt; cloroperone, in particular the hydrochloride salt; clothiapine; clothixamide, in particular the maleate salt; clozapine; cyclophenazine, in particular the hydrochloride salt; droperidol; etazolate, in particular the hydrochloride salt; fenimide; flucindole; flumezapine; fluphenazine, in particular the decanoate, enanthate and/or hydrochloride salts; fluspiperone; fluspirilene; flutroline; gevotroline, in particular the hydrochloride salt; halopemide; haloperidol; iloperidone; imidoline, in particular the hydrochloride salt; lenperone; loxapine; mazapertine, in particular the succinate salt; mesoridazine; metiapine; milenperone; milipertine; molindone, in particular the hydrochloride salt; naranol, in particular the hydrochloride salt; neflumozide, in particular the hydrochloride salt; ocaperidone; olanzapine; oxiperomide; penfluridol; pentiapine, in particular the maleate salt; perphenazine; pimozide; pinoxepin, in particular the hydrochloride salt; pipamperone; piperacetazine; pipotiazine, in particular the palmitate salt; piquindone, in particular the hydrochloride salt; prochlorperazine, in particular the edisylate salt; prochlorperazine, in particular the maleate salt; promazine, in particular the hydrochloride salt; quetiapine; remoxipride; risperidone; rimcazol, in particular the hydrochloride salt; seperidol, in particular the hydrochloride salt; sertindole; setoperone; spiperone; sulpiride; thioridazine; thiothixene; thorazine; tioperidone, in particular the hydrochloride salt; tiospirone, in particular the hydrochloride salt; trifluoperazine, in particular the hydrochloride salt; trifluperidol; triflupromazine; ziprasidone, in particular the hydrochloride salt; and mixtures thereof.

Some compound according to the invention surprisingly also shows a moderate 5-HT-reuptake inhibition activity and may therefore very well be suited for use in the treatment and/or prophylaxis of depression. It is thought that a 5-HT reuptake inhibitor with associated α₂-adrenoceptor antagonistic activity might be a new type of antidepressant, with a dual action on the central noradrenergic and serotonergic neuronal systems. The immediate effect on monoamine release of autoreceptor blockade may accelerate the onset of action of such a compound, compared to currently available drugs that require desensitization of the autoreceptors involved in the feedback mechanism in order to become fully effective. In addition, α_{2C}-adrenoceptor antagonism improves sexual function as shown by treatment with the α_{2C}-adrenoceptor antagonist yohimbine, thereby potentially reducing one of the side effects related to 5-HT uptake inhibition and enhancement of NEergic neurotransmission improves social function more effectively than SSRIs (J. Ignacio Andrés et al., J. Med. Chem. (2005), Vol. 48, 2054-2071)).

### Pharmaceutical compositions

The invention also relates to a pharmaceutical composition comprising a pharmaceutically acceptable carrier or diluent and, as active ingredient, a therapeutically effective amount of a compound according to the invention, in particular compound according to Formula (I), a pharmaceutically acceptable acid or base addition salt thereof, an N-oxide form thereof, or a quaternary ammonium salt thereof.

A compound according to the invention or any subgroup or combination thereof may be formulated into various pharmaceutical forms for administration purposes. As appropriate compositions there may be cited all compositions usually employed for systemically administering drugs.

To prepare the pharmaceutical composition of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. This pharmaceutical composition is desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. examples of such unit dosage forms are tablets (including scored or coated tablets), capsules, pills, powder packets, wafers, suppositories, injectable solutions or suspensions and the like, and segregated multiples thereof. Since a compound according to the invention is a potent orally administrable dopamine antagonist, a pharmaceutical composition comprising said compound for administration orally is especially advantageous.

The invention also relates to a pharmaceutical composition comprising a compound according to the invention and one or more other compounds selected from the group of antidepressants, anxiolytics and antipsychotics as well as to the use of such a composition for the manufacture of a medicament, in particular to improve efficacy and/or onset of action in the treatment of depression and/or anxiety.

The following examples are intended to illustrate but not to limit the scope of the present invention.

### Experimental part

Hereinafter, "THF" means tetrahydrofuran ; "DMF" means *N*,*N*-dimethylformamide ; "EtOAc" means ethyl acetate ; "DMSO" means dimethylsulfoxide ; "DCM" means dichloromethane; "1,2-DCE" means 1,2-dichloroethane "HPLC" means High-performance liquid chromatography; "PS-TBD" is a polymer-supported base which consists of a bicyclic guanidine moiety (1,5,7-triazabicyclo[4.4.0]dec-5-ene) anchored on polystyrene and "PS-NCO" means polymer-supported isocyanate.

Microwave assisted reactions were performed in a single-mode reactor: Emrys^{™} Optimizer microwave reactor (Personal Chemistry A.B., currently Biotage). Description of the instrument can be found in www.personalchemistry.com. And in a multimode reactor: MicroSYNTH Labstation (Milestone, Inc.). Description of the instrument can be found in www.milestonesci.com.

### A. Preparation of the intermediate compounds

a) Preparation of intermediate compound I-1
   a1) Preparation of (1-benzyl-piperidin-4-yl)-(3-chloro-pyrazin-2-yl)-amine 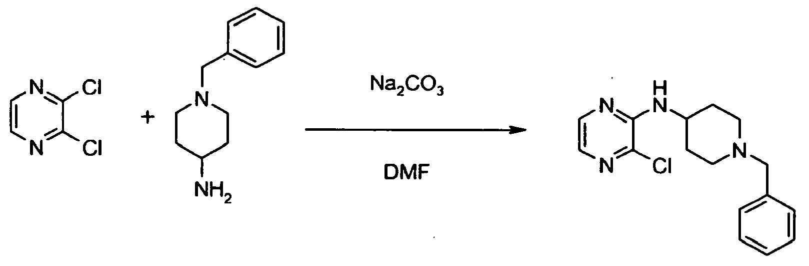 2,3-Dichloropyrazine (10 g, 62.12 mmol) and 1-(benzyl)-4-piperidinamine (13.73 mL, 67.12 mmol) were dissolved in DMF (60 ml). Then Na₂CO₃ (10.09 g, 114.10 mmol) was added. The reaction was stirred at 130 °C for 16 hours. The solid was filtered off, washed with EtOAc and the solvent was evaporated till dryness. The product was dissolved in EtOAc, washed with H₂O and brine, dried with MgSO₄ and evaporated under vacuum. The product was used without any further purification yielding 15 g of (1-benzyl-piperidin-4-yl)-(3-chloro-pyrazin-2-yl)-amine (74 %).
   a2) Preparation of 3-(1-benzyl-piperidin-4-ylamino)-1H-pyrazin-2-one 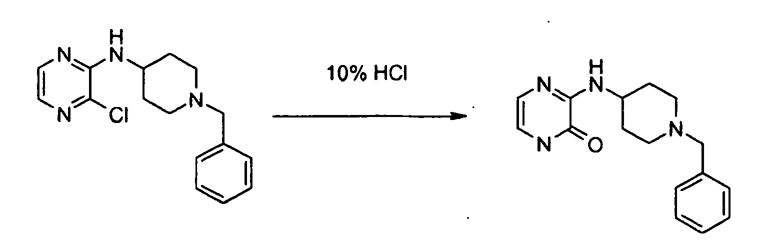 (1-Benzyl-piperidin-4-yl)-(3-chloro-pyrazin-2-yl)-amine, obtained in step a1 (7 g, 23.11 mmol) was dissolved in HCI (70 ml; 10 %) and heated in a sealed tube at 110 °C for 16 hours. A light brown solid was precipitated, it was filtered off, washed with water and dried under vacuum yielding 4.57 g of 3-(1-benzyl-piperidin-4-ylamino)-1H-pyrazin-2-one (70 %).
   a3) Preparation of intermediate compound I-1 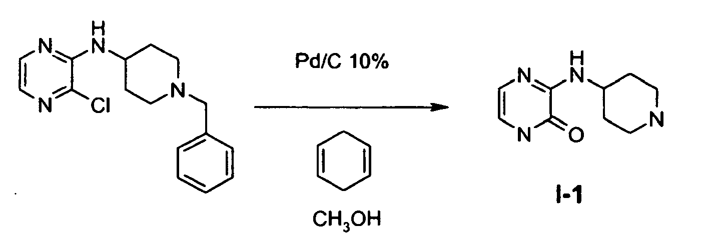 3-(1-Benzyl-piperidin-4-ylamino)-1H-pyrazin-2-one, obtained in step a2 (4.17 g, 14.66 mmol) was dissolved in CH₃OH (62 mL), then Pd/C (4.17 g; 10 %) and 1,4-cyclohexadiene (27.96 mL, 293.2 mmol) were added. The reaction was heated in a sealed tube at 65 °C for 4 hours. The reaction was filtered over celite and the solvent was evaporated till dryness yielding 2.69 g of the desired intermediate compound I-1 (94 %).
b) Preparation of intermediate compound I-2 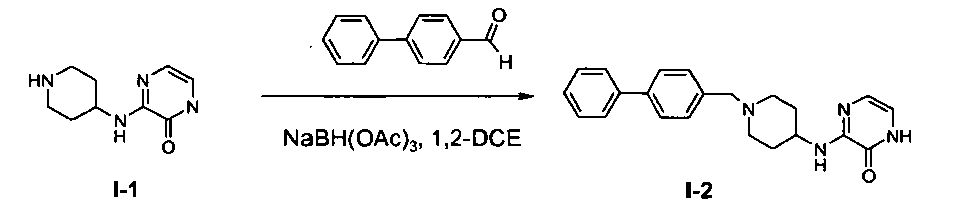 Intermediate compound I-1 (5.15 mmol), 4-biphenylcarboxaldehyde (2.81 g, 15.45 mmol) and sodium triacetoxyborohydride (1.63 g, 7.72 mmol) were solved in 1,2-DCE (30 ml). The reaction was stirred at room temperature for 16 h. The solvent was concentrated. The residue was solved in EtOAc and washed with Na₂CO₃ aqueous saturated solution, and NaCl aqueous saturated solution. The combined organic phases were dried (MgSO4), filtered and concentrated. The residue was purified by catch and release using a cartridge of isolute SCX-3, in MeOH, and then MeOH/NH3. The ammonia phase was concentrated. The residue was purified by chromatography with SiO₂ (eluent DCM-MeOH(NH₃) 9-1 to 7-3) to yield intermediate I-2 (0.949 g, 51 %).
b) Preparation of intermediate compound I-3 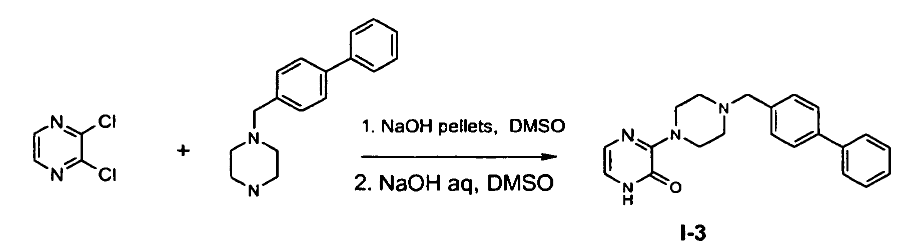 2,3-Dichloropyrazine (180 mg, 0.117 mmol), 1-([1,1'-biphenyl]-4-ylmethyl)piperazine (CAS 84359-51-3, 0.0981 mmol) and NaOH pellets (400 mg) were heated at 150 °C in a sealed tube during 4h.
   Then 0.4 ml of NaOH 4 M and 0.4 ml of DMSO were added, heating at 150 °C for 5 hours more. The crude was extracted with DCM and water. The combined organic phases were dried (MgSO4), filtered and concentrated. The residue was purified by chromatography in SiO₂ to obtain intermediate compound I-3 which was used in next reaction step without further purification.
c) Preparation of intermediate compound I-4 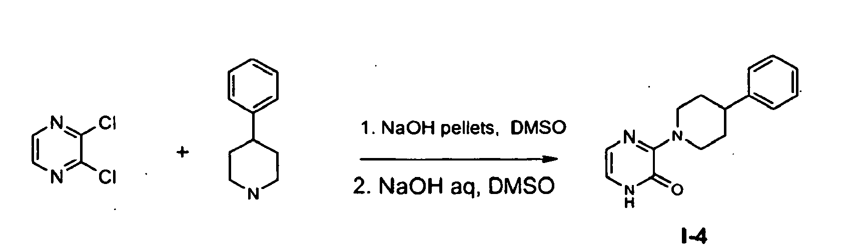 2,3-Dichloropyrazine (448 mg, 3 mmol) and 4-phenylpiperidine (CAS: 771-99-3, 2.9 mmol) were dissolved in DMSO (0.400 ml). Then NaOH pellets (1 g, 25 mmol) were added. The reaction was stirred at 150 °C under microwave irradiation for 0.5 hours. Then 0.4 ml of NaOH 4 M and 0.4 ml of DMSO were added, heating at 150 °C in microwave for 0.5 hours more. The mixture was dissolved in EtOAc, washed with H₂O and brine, dried (MgSO₄) and evaporated . The residue was purified in a short open column chromatography of SiO₂, to yield intermediate I-4 which was used in next reaction step without further purification.
d) Preparation of intermediate compound I-5 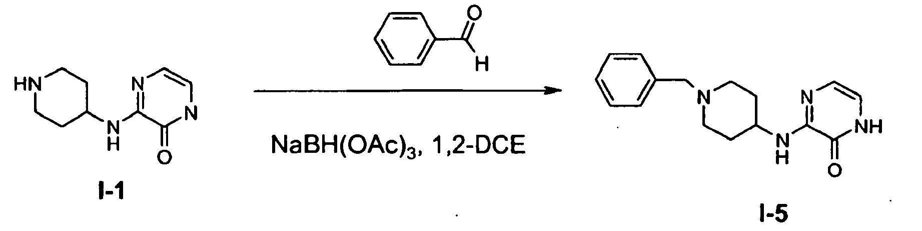 Compound I-1 (5.15 mmol), benzaldehyde (2.81 g, 15.45 mmol) and sodium triacetoxyborohydride (1.63 g, 7.72 mmol) were solved in 1,2-DCE (30 ml). The reaction was stirred at room temperature for 16 h. The solvent was concentrated. The residue was solved in EtOAc and washed with NaHCO₃ aqueous saturated solution, and NaCl aqueous saturated solution. The combined organic phases were dried (MgS04), filtered and concentrated. The residue was purified by catch and release using a cartridge of isolute SCX-3, in MeOH, and then MeOH/NH3. The ammonia phase was concentrate to yield intermediate I-5 (1.30 g, 89 %)
e) Preparation of intermediate compound I-6 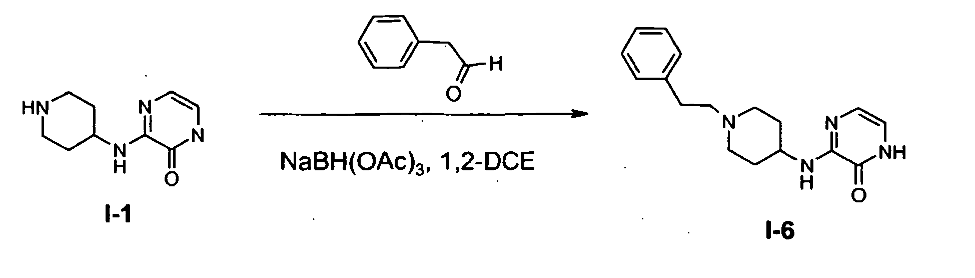 Compound I-1 (1 g, 5.15 mmol), phenylacetaldehyde (CAS: 122-78-1, 1.81 ml, 15.45 mmol) and sodium triacetoxyborohydride (1.63 g, 7.72 mmol) were solved in 1,2-DCE (30 ml). The reaction was stirred at room temperature for 16 h. The solvent was concentrated. The residue was solved in EtOAc and washed with NaHCO₃ aqueous saturated solution, and NaCl aqueous saturated solution. The combined organic phases were dried (MgSO4), filtered and concentrated, to yield intermediate I-6 (1.07g, 71 %).
f) Preparation of intermediate compound 1-7 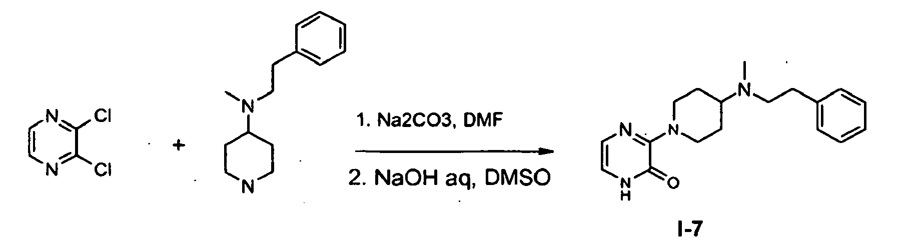 2,3-Dichloropyrazine (1 g, 4.5 mmol), N-methyl-N-(2-phenylethyl) 4 piperidinamine (CAS: 142752-20-3, 4.5 mmol) were dissolved in DMF (7.5 ml). Then Na₂CO₃ (0.84 g, 7.9 mmol) were added. The reaction was stirred at 130 °C for 5 hours. The reaction mixture was washed with H₂O and extracted with DCM. The combined organic phases were dried (Na₂SO4), filtered and the solvent concentrated, to yield 1.2 g. This compound was suspended in a mixture of NaOH 4 M (6 ml) and 6 ml of DMSO, heating at 150 °C in microwave for 0.5 hours. The mixture was dissolved in EtOAc, washed with NH₄Cl (10 %). The combined organic phases were dried with MgSO₄ and evaporated to yield intermediate compound I-7 (0.82 g, 58 %)
g) Preparation of intermediate compound I-8 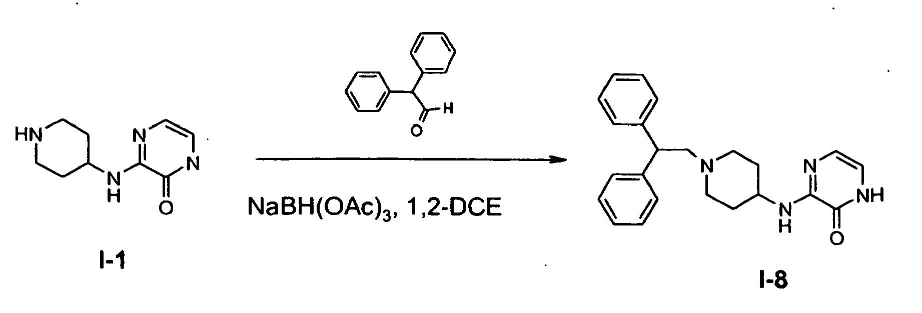 Compound I-1 (0.6 g, 3.09 mmol), diphenylacetaldehyde (CAS: 947-91-1, 9.27 mmol) and sodium triacetoxyborohydride (0.982 g, 4.63 mmol) were solved in 1,2-DCE (30 ml). The reaction was stirred at room temperature for 16 h. The solvent was concentrated. The residue was solved in EtOAc and washed with NaHCO₃ aqueous saturated solution, and NaCl aqueous saturated solution. The combined organic phases were dried (MgSO4), filtered and concentrated. The residue was purified by chromatography with SiO₂ (eluent DCM/MeOH(NH₃) 9.5/0.5) to yield intermediate I-8 (0.867 g, 75 %) as a solid.
h) Preparation of intermediate compound 1-9 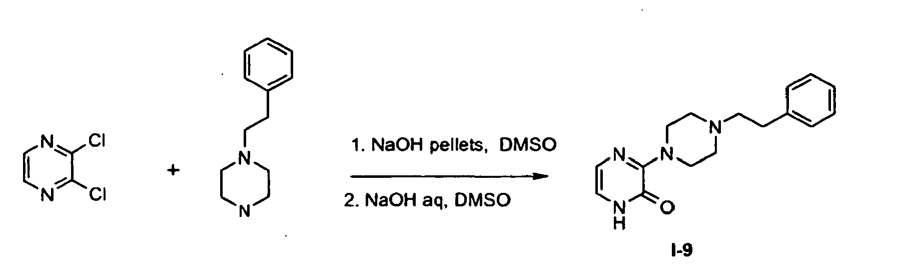 2,3-Dichloropyrazine (448 mg, 3 mmol) and 1-(2-phenylethylypiperazine (CAS: 5321-49-3, 2.9 mmol) were dissolved in DMSO (0.400 ml). Then NaOH pellets (1 g, 25 mmol) were added. The reaction was stirred at 150 °C under microwave irradiation for 0.5 hours.
   Then 0.4 ml of NaOH 4 M and 0.4 ml of DMSO were added, heating at 150 °C in microwave for 0.5 hours more. The mixture was dissolved in EtOAc, washed with H₂O and brine, dried (Na₂SO₄) and evaporated .The residue was purified in a short open column chromatography of SiO₂, to yield intermediate I-9 which was used in next reaction step without further purification.
i) Preparation of intermediate compound I-10 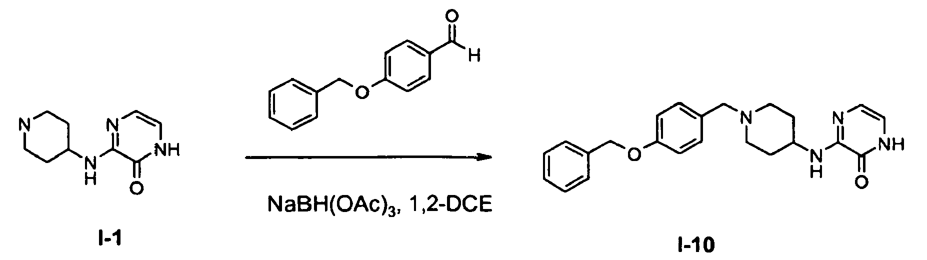 Compound I-1 (1.2 g, 6.18 mmol), 4-benzyloxybenzaldehyde (CAS: 4397-53-9, 18.53 mmol) and sodium triacetoxyborohydride (1.96 g, 9.27 mmol) were solved in 1,2-DCE (30 ml). The reaction was stirred at room temperature for 64 h. The solvent was concentrated. The residue was solved in EtOAc and washed with NaHCO₃ aqueous saturated solution, and NaCl aqueous saturated solution. The combined organic phases were dried (MgSO4), filtered and concentrated. The residue was purified by flash chromatography with SiO2 (eluent DCM/MeOH 95 /5) to yield intermediate I-10 (2.05 g, 85 %) as a solid.

### B. Preparation of the final compounds

a) Preparation of final compound 1-3 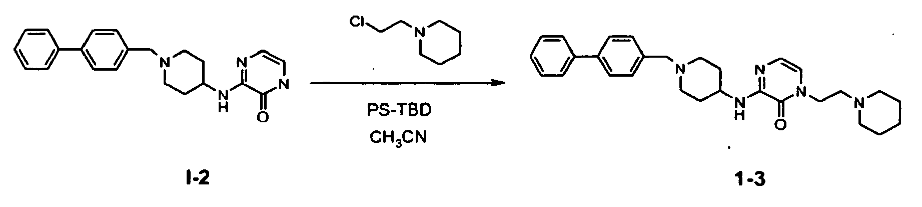 Intermediate compound **I-2** (20 mg, 0.055 mmol), 1-(2-chloroethyl)piperidine monohydrochloride (0.16 mmol) and PS-TBD (62 mg, 0.16 mmol) were suspended in CH₃CN (1 ml). The reaction was heated in the microwave at 120 °C for 20 minutes. The resin was filtered off, and the filtrate was concentrated under vacuum. The resulting crude was purified by catch and realease using a SCX-2 cartridge yielding 0.0115 g of the purified final compound **1-3** (60 %).
b) Preparation of final compound 2-2 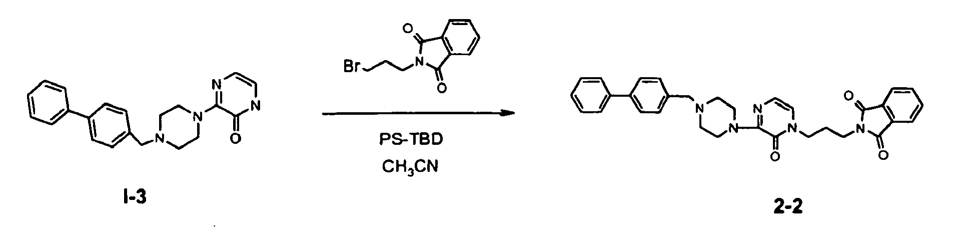 Intermediate compound **I-3** (72 mg, 0.2 mmol), N-(3-bromopropyl)phthalimide (0.4 mmol) and PS-TBD (207 mg, 0.6 mmol) were suspended in CH₃CN (2 ml). The reaction was heated in the microwave at 120 °C for 15 minutes. The resin was filtered off, and the filtrate was concentrated under vacuum. The resulting crude was purified by HPLC yielding 0.037g of the purified final compound 2-2 (35 %).
c) Preparation of final compound 3-3 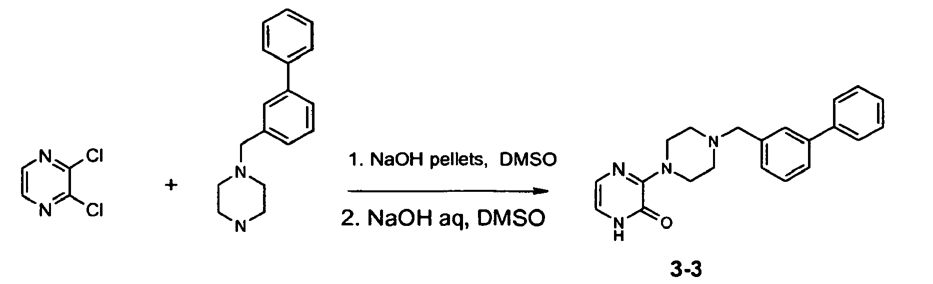 2,3-Dichloropyrazine (500 mg, 3.35 mmol), 1-([1,1'-biphenyl]-3-ylmethyl) piperazine, (CAS 204634-72-0, 3.2 mmol) and NaOH pellets (1 g, 25 mmol) were heated at 150 °C in a sealed tube during 4h.
   Then 0.4 ml of NaOH 4 M and 0.4 ml of DMSO were added, heating at 150 °C for 5 hours more. The crude was extracted with DCM and water. The combined organic phases were dried (MgSO4), filtered and concentrated. The residue was purified by chromatography in SiO₂ to obtain final compound 3-3 (0.730 g, 63 %).
d) Preparation of final compound 3-5 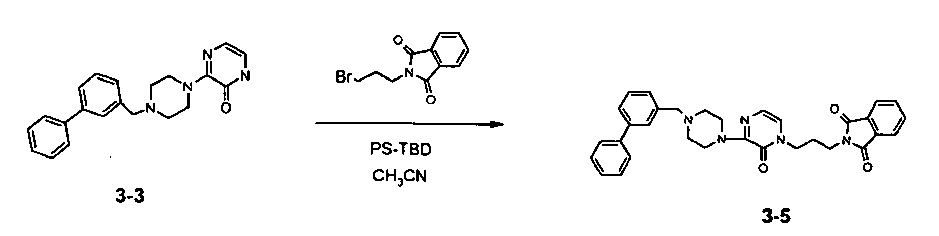 Final compound **3-3** (69 mg, 0.2 mmol), N-(3-bromopropyl)phthalimide (0.4 mmol) and PS-TBD (207 mg, 0.6 mmol) were suspended in CH₃CN (2 ml). The reaction was heated in the microwave at 120 °C for 15 minutes. The resin was filtered off, and the filtrate was concentrated under vacuum. The resulting crude was purified by HPLC yielding 0.043 g of the purified final compound 3-5 (40 %).
e) Preparation of final compound 4-1 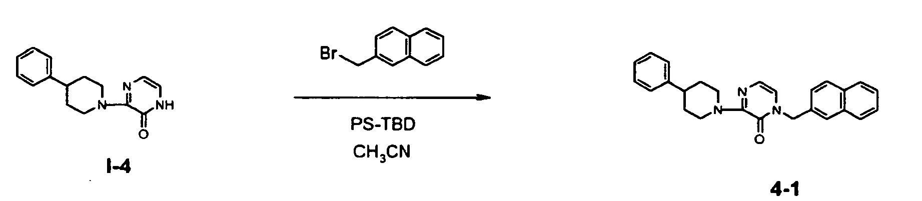 Intermediate compound **I-4** (25 mg, 0.1 mmol), 2-(bromomethyl)naphthalene (0.2 mmol) and PS-TBD (103 mg, 0.3 mmol) were suspended in CH₃CN (2 ml). The reaction was heated in the microwave at 120 °C for 20 minutes. The resin was filtered off, and the filtrate was concentrated under vacuum. The resulting crude was purified by HPLC yielding 0.028 g of the purified final compound **4-1** (65 %).
f) Preparation of final compound 5-5 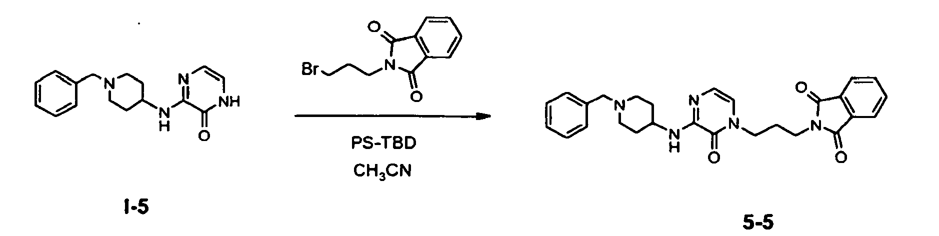 Intermediate compound **I-5** (20 mg, 0.07 mmol), N-(3-bromopropyl)phthalimide (0.21 mmol) and PS-TBD (76 mg, 0.21 mmol) were suspended in CH₃CN (1 ml). The reaction was heated in the microwave at 120 °C for 20 minutes. The resin was filtered off, and the filtrate was concentrated under vacuum. The resulting crude was purified by catch and realease using a SCX-2 cartridge, yielding 0.017 g of the purified final compound 5-5 (53 %).
g) Preparation of final compound 6-6 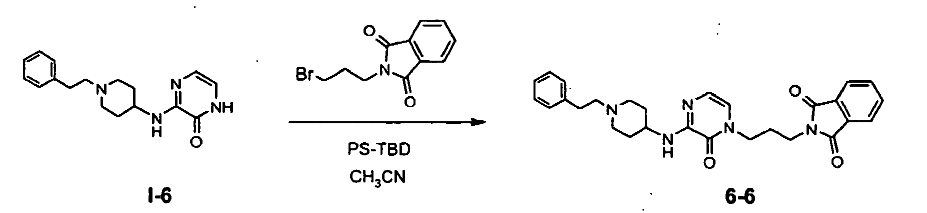 Intermediate compound **I-6** (20 mg, 0.067 mmol), N-(3-bromopropyl)phthalimide (0.20 mmol) and PS-TBD (69 mg, 0.20 mmol) were suspended in CH₃CN (1 ml). The reaction was heated in the microwave at 120 °C for 20 minutes. The resin was filtered off, and the filtrate was concentrated under vacuum. The resulting crude was purified by by catch and realease using a SCX-2 cartridge, yielding 0.014 g of the purified final compound **6-6** (43 %).
h) Preparation of final compound 7-5 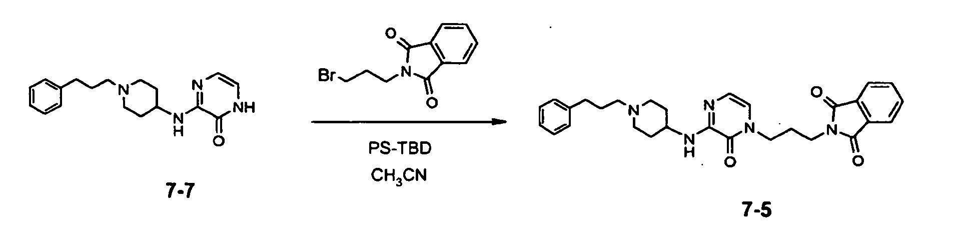 Final compound **7-7** (20 mg, 0.064 mmol), N-(3-bromopropyl)phthalimide (0.192 mmol) and PS-TBD (71 mg, 0.192 mmol) were suspended in CH₃CN (1 ml). The reaction was heated in the microwave at 130 °C for 20 minutes. The resin was filtered off, and the filtrate was concentrated under vacuum. The resulting crude was purified by by catch and realease using a SCX-2 cartridge, yielding 0.0122 g of the purified final compound **7-5** (36 %).
i) Preparation of final compound 7-7 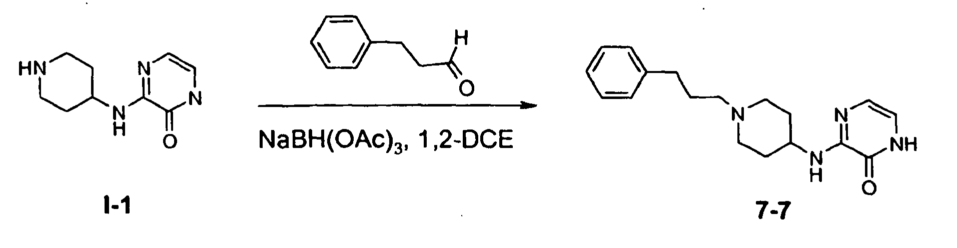 Compound I-1 (5.15 mmol), hydrocinnamaldehyde (CAS: 104-53-0, 2.81 g, 15.45 mmol) and sodium triacetoxyborohydride (1.63 g, 7.72 mmol) were solved in 1,2-DCE (30 ml). The reaction was stirred at room temperature for 16 h. The solvent was concentrated. The residue was solved in EtOAc and washed with NaHCO₃ aqueous saturated solution, and NaCl aqueous saturated solution. The combined organic phases were dried (MgSO4), filtered and concentrated. The residue was purified by catch and released using a cartridge of isolute SCX-3, in MeOH, and then MeOH/NH₃. The ammonia phase was concentrated to yield compound 7-7 (1.30 g, 89 %)
j) Preparation of final compound 8-1 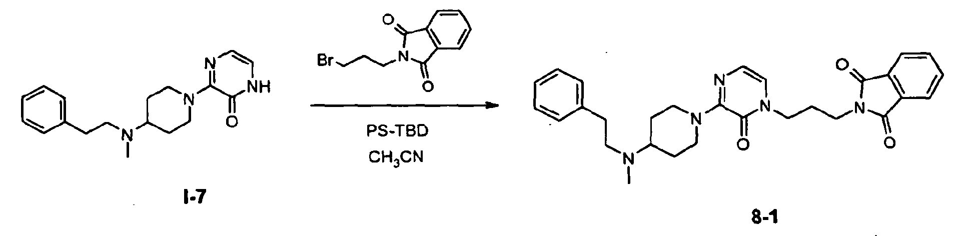 Intermediate compound **I-7** (50 mg, 0.160 mmol), N-(3-bromopropyl)phthalimide (0.240 mmol) and PS-TBD (160 mg, 0.48 mmol) were suspended in CH₃CN (2.5 ml). The reaction was heated in the microwave at 120 °C for 20 minutes. The resin was filtered off, and the filtrate was concentrated under vacuum. The resulting crude was purified by HPLC yielding 0.038 g of the purified final compound **8-1** (48 %).
k) Preparation of final compound 9-3 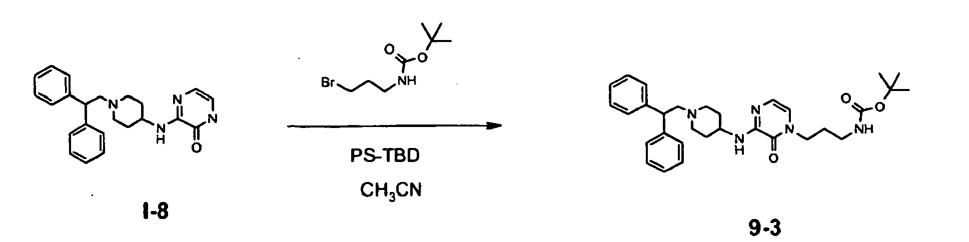 Intermediate compound **I-8** (20 mg, 0.053 mmol), 3-(boc-amino)propyl bromide (0.160 mmol) and PS-TBD (55 mg, 0.160 mmol) were suspended in CH₃CN (1 ml). The reaction was heated in the microwave at 120 °C for 20 minutes. The resin was filtered off, and the filtrate was concentrated under vacuum. The resulting crude was purified by catch and release using a SCX-2 cartridge to yield 0.010 g of the purified final compound **9-3** (37 %).
l) Preparation of final compound 9-3 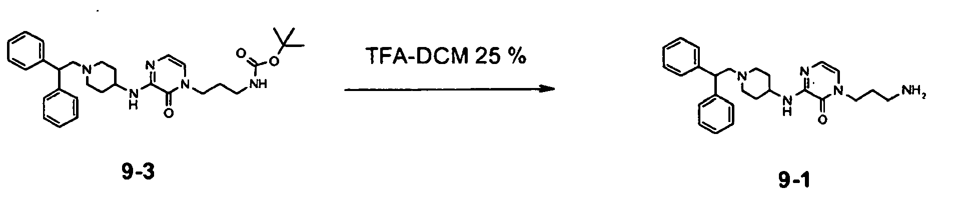 Final compound **9-3** (15 mg, 0.028 mmol) was solved in a mixture of TFA-DCM 25 % (1 ml). The reaction was stirred at room temperature for 1 h. The solvent was concentrated. The crude was solved in MeOH and purified by catch and release using a SCX-2 cartridge, to yield 10 mg (84 %) of compound **9-1.**
m) Preparation of final compound 10-11 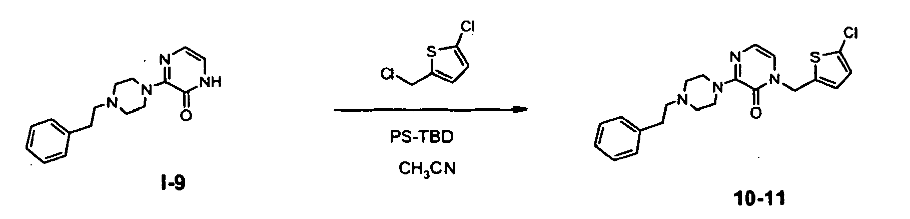 Intermediate compound **I-9** (28 mg, 0.1 mmol), 2-chloro-5-(chloromethyl)thiophene (0.2 mmol) and PS-TBD (103 mg, 0.3mmol) were suspended in CH₃CN (2 ml). The reaction was heated in the microwave at 120 °C for 20 minutes. The resin was filtered off, and the filtrate was concentrated under vacuum. The resulting crude was purified by HPLC yielding 0.031 g of the purified final compound **10-11** (75 %).
n) Preparation of final compound 11-5 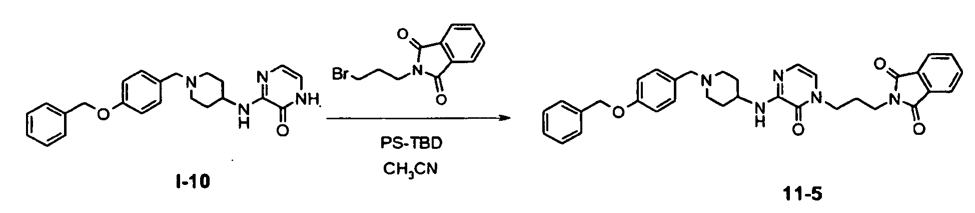 Intermediate compound **I-10** (29 mg, 0.073 mmol), *N*-(3-bromopropyl)phthalimide (0.22 mmol) and PS-TBD (76 mg, 0.22 mmol) were suspended in CH₃CN (2 ml). The reaction was heated in the microwave at 120 °C for 15 minutes. The resin was filtered off, and the filtrate was concentrated under vacuum. The resulting crude was purified by catch and release with a SCX-2 cartridge, to yield 0.023 g of the purified final compound **11-5** (55 %).

The following compounds were prepared according to the above examples, schemes and procedures.

**Table 1: List of biphenyl-substituted compounds (with amino-piperidinyl-linker).**

| | | | |
|---|---|---|---|
| 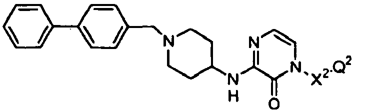 | | | |

| **Co.Nr.** | **Scheme** | **--X²--** | **--Q²** |
|---|---|---|---|
| **1-1** | 3A | 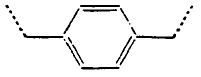 | -NH₂ |
| **1-2** | 2A | 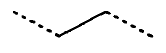 | 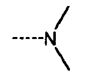 |
| **1-3** | 2A | 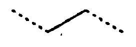 | 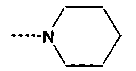 |
| **1-4** | 2A | 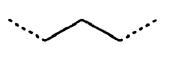 | 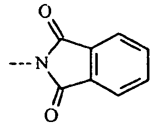 |
| **1-5** | 2A | 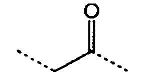 | --OCH₃ |
| **1-6** | 2A | --CH₂-- | 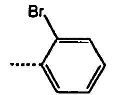 |
| **1-7** | 2A | --CH₂-- | 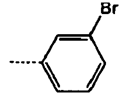 |
| **1-8** | 2A | --CH₂-- | 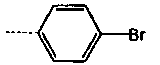 |
| **1-9** | 2A | --CH₂-- | 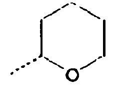 |
| **1-10** | 2A | --CH₂-- | 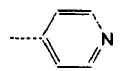 |
| **1-11** | 2A | --CH₂-- | 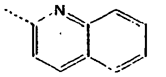 |

**Table 2: List of biphenyl-substituted compounds (with piperazinyl-linker).**

| | | | |
|---|---|---|---|
| 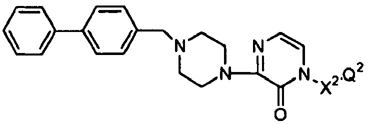 | | | |

| **Co.Nr.** | **Scheme** | **--X²--** | **--Q²** |
|---|---|---|---|
| **2-1** | 2A | 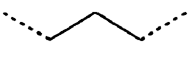 | 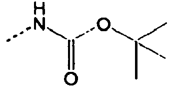 |
| **2-2** | 2A | 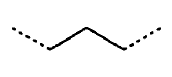 | 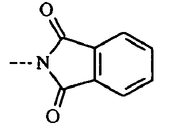 |

**Table 3: List of biphenyl substituted compounds (with piperazinyl-linker).**

| | | | |
|---|---|---|---|
| 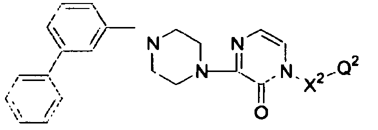 | | | |

| **Co.Nr.** | **Scheme** | **--X²--** | **--Q²** |
|---|---|---|---|
| **3-1** | 3A | 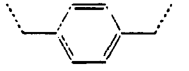 | -NH₂ |
| **3-2** | 2A | 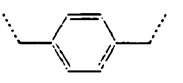 | 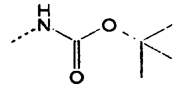 |
| **3-3** | 1A | cb | H |
| **3-4** | 2A | 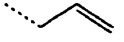 | H |
| **3-5** | 2A | -CH₂- | 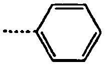 |
| **3-6** | 2A | 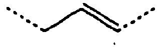 E+Z | 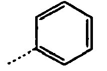 |
| **3-7** | 2A | -CH₂-- | 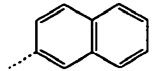 |
| **3-8** | 2A | --CH₂-- | 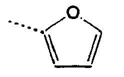 |
| **3-9** | 2A | 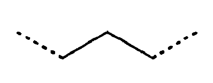 | 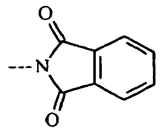 |
| **3-10** | 2A | --CH₂-- | 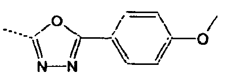 |

**Table 4: List of phenyl-substituted compounds (with piperidinyl-linker)**

| | | | |
|---|---|---|---|
| 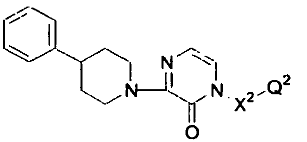 | | | |

| **Co.Nr.** | **Scheme** | **--X²--** | **--Q²** |
|---|---|---|---|
| **4-1** | 2A | --CH₂-- | 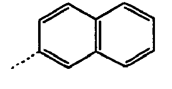 |
| **4-2** | 3A | 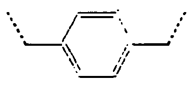 | -NH₂ |
| **4-3** | 2A | 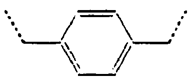 | 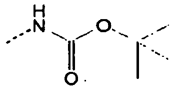 |

**Table 5: List of phenyl-substituted compounds (with piperidinylamino-linker)**

| | | | | |
|---|---|---|---|---|
| 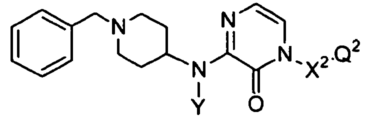 | | | | |

| **Co. Nr.** | **Scheme** | **--Y--** | **--X²-** | **--Q²** |
|---|---|---|---|---|
| **5-1** | 3A | -H | 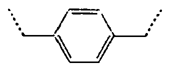 | -NH₂ |
| **5-2** | 3A | -H | 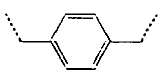 | -NH₂ |
| **5-3** | 3A | 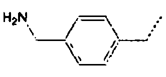 | 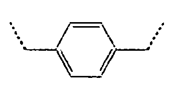 | -NH₂ |
| **5-4** | 2A | -H | 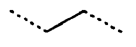 | 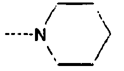 |
| **5-5** | 2A | -H | 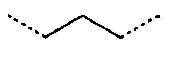 | 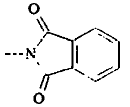 |

**Table 6: List of phenyl-substituted compounds (with piperidinylamino-linker).**

| | | | |
|---|---|---|---|
| 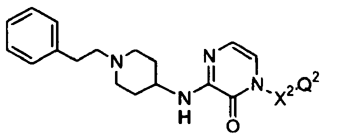 | | | |

| **Co.Nr.** | **Scheme** | **--X²--** | **--Q²** |
|---|---|---|---|
| **6-1** | 3A | 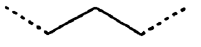 | -NH₂ |
| **6-2** | 3A | 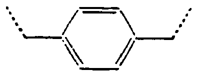 | -NH₂ |
| **6-3** | 3A | 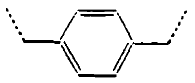 | -NH₂ |
| **6-4** | 2A | 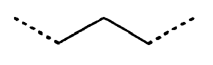 | 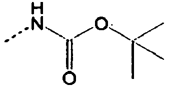 |
| **6-5** | 2A | 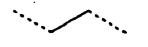 | 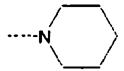 |
| **6-6** | 2A | 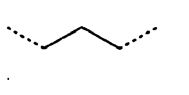 | 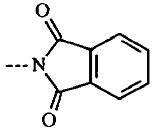 |
| **6-7** | 2A | --CH₂-- | 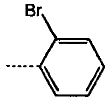 |
| **6-8** | 2A | --CH₂-- | 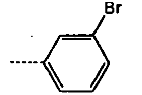 |
| **6-9** | 2A | --CH₂-- | 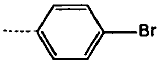 |

**Table 7: List of phenyl-substituted compounds (with piperidinylamino-linker).**

| | | | |
|---|---|---|---|
| 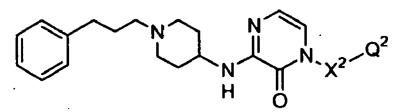 | | | |

| **Co.Nr.** | **Scheme** | --X²-- | **--Q²** |
|---|---|---|---|
| **7-1** | 3A | 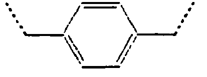 | -NH₂ |
| **7-2** | 2A | 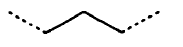 | 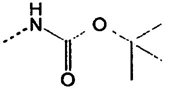 |
| **7-3** | 2A | 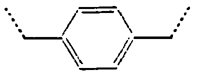 | 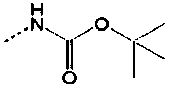 |
| **7-4** | 2A | 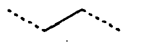 | 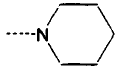 |
| **7-5** | 2A | 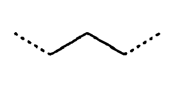 | 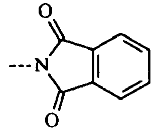 |
| **7-6** | 2A | 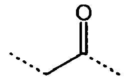 | -OCH₃ |
| **7-7** | 1B | cb | H |
| **7-8** | 2A | 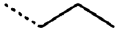 | H |
| **7-9** | 2A | 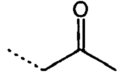 | H |
| **7-10** | 2A | 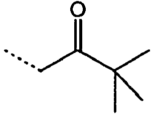 | H |
| **7-11** | 2A | --CH₂-- | 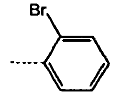 |
| **7-12** | 2A | --CH₂-- | 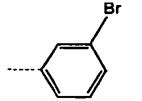 |
| **7-13** | 2A | --CH₂-- | 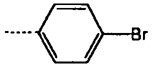 |
| **7-14** | 2A | 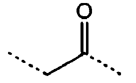 | 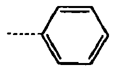 |
| **7-15** | 2A | 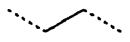 | S-CH₃ |

**Table 8: List of phenyl-substituted compounds (with aminopiperidinyl-linker).**

| | | | |
|---|---|---|---|
| 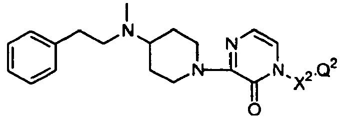 | | | |

| **Co.Nr.** | **Scheme** | **--X²--** | **--Q²** |
|---|---|---|---|
| **8-1** | 2A | 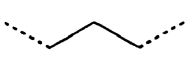 | 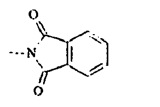 |
| **8-2** | 2A | 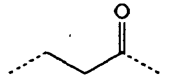 | 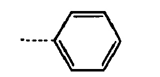 |

**Table 9: List of 1,1-diphenylmethyl-substituted compounds (with piperidinylamino-linker).**

| | | | |
|---|---|---|---|
| 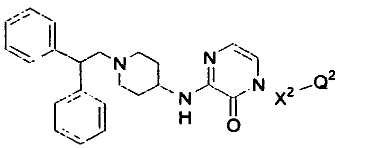 | | | |

| **Co.Nr.** | **Scheme** | **--X²--** | **--Q²** |
|---|---|---|---|
| **9-1** | 3A | 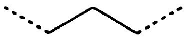 | -NH₂ |
| **9-2** | 3A | 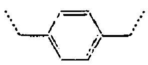 | -NH₂ |
| **9-3** | 2A | 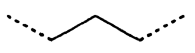 | 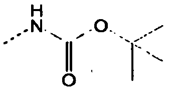 |
| **9-4** | 2A | 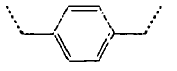 | 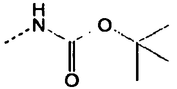 |
| **9-5** | 2A | 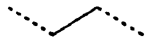 | 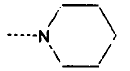 |
| **9-6** | 2A | 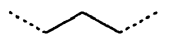 | 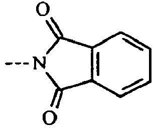 |
| **9-7** | 2A | --CH₂-- | 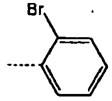 |
| **9-8** | 2A | -CH₂- | 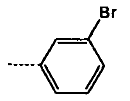 |
| **9-9** | 2A | --CH₂-- | 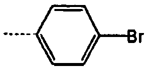 |
| **9-10** | 2A | 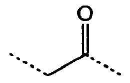 | 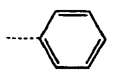 |

**Table 10 : List of phenyl-substituted compounds (with piperazinyl-linker).**

| | | | | |
|---|---|---|---|---|
| 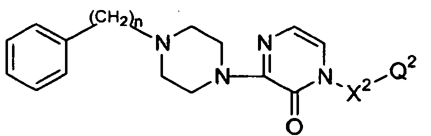 | | | | |

| **Co.Nr.** | **Scheme** | **n** | **--X²--** | **--Q²** |
|---|---|---|---|---|
| **10-1** | 3A | 2 | 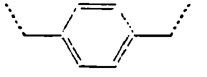 | -NH₂ |
| **10-2** | 2A | 1 | 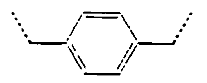 | 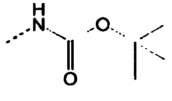 |
| **10-3** | 2A | 2 | 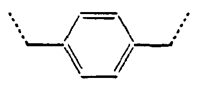 | 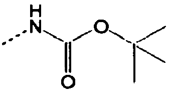 |
| **10-4** | 2A | 2 | 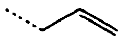 | H |
| **10-5** | 2A | 1 | 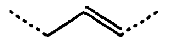 E+Z | 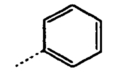 |
| **10-6** | 2A | 2 | 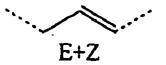 | 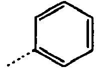 |
| **10-7** | 2A | 2 | 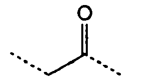 | 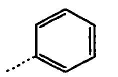 |
| **10-8** | 2A | 2 | 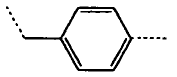 | 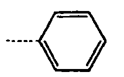 |
| **10-9** | 2A | 1 | --CH₂-- | 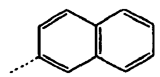 |
| **10-10** | 2A | 2 | --CH₂-- | 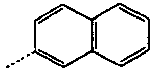 |
| **10-11** | 2A | 2 | --CH₂-- | 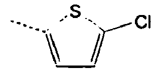 |
| **10-12** | 2A | 2 | -CH₂-- | 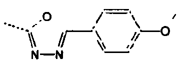 |

**Table 11: List of phenyl-substituted compounds (with piperidinylamino-linker).**

| | | | |
|---|---|---|---|
| 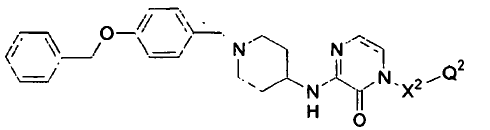 | | | |

| **Co.Nr.** | **Scheme** | **--X²--** | **--Q²** |
|---|---|---|---|
| **11-1** | 3A | 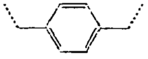 | -NH₂ |
| **11-2** | 2A | 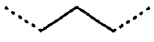 | 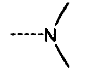 |
| **11-3** | 2A | 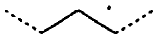 | 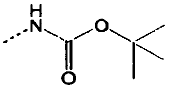 |
| **11-4** | 2A | 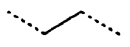 | 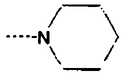 |
| **11-5** | 2A | 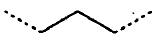 | 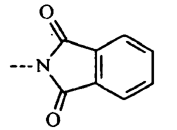 |
| **11-6** | 2A | --CH₂-- | 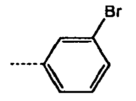 |
| **11-7** | 2A | --CH₂-- | 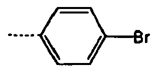 |

### C. Pharmacological example

### General

The interaction of a compound of Formula (I) with α_{2C}-adrenoceptor receptors was assessed in *in vitro* radioligand binding experiments. In general, a low concentration of a radioligand with a high binding affinity for a particular receptor or transporter is incubated with a sample of a tissue preparation enriched in a particular receptor or transporter or with a preparation of cells expressing cloned human receptors in a buffered medium. During the incubation, the radioligand binds to the receptor or transporter. When equilibrium of binding is reached, the receptor bound radioactivity is separated from the non-bound radioactivity, and the receptor- or transporter-bound activity is counted. The interaction of the test compounds with the receptor is assessed in competition binding experiments. Various concentrations of the test compound are added to the incubation mixture containing the receptor- or transporter preparation and the radioligand. The test compound in proportion to its binding affinity and its concentration inhibits binding of the radioligand. The radioligand used for hα_{2A} and hα_{2C} receptor binding was [³H]-raulwolscine.

### Example C.1 : Binding Experiment for α_{2C}-adrenoceptor

### Cell culture and membrane preparation.

CHO cells, stabile transfected with human adrenergic-α_{2A} and α_{2C} receptor cDNA, were cultured in Dulbecco's Modified Eagle's Medium (DMEM)/Nutrient mixture Ham's F12 (ratio 1:1)(Gibco, Gent-Belgium) supplemented with 10 % heat inactivated fetal calf serum (Life Technologies, Merelbeke-Belgium) and antibiotics (100 IU/ml penicillin G, 100 µg/ml streptomycin sulphate, 110 µg/ml pyruvic acid and 100 µg/ml L-glutamine). One day before collection, cells were induced with 5 mM sodiumbutyrate. Upon 80-90 % of confluence, cells were scraped in phosphate buffered saline without Ca²⁺ and Mg²⁺ and collected by centrifugation at 1500 x g for 10 minutes. The cells were homogenised in Tris-HCl 50 mM using an Ultraturrax homogenizer and centrifuged for 10 minutes at 23,500 x g. The pellet was washed once by resuspension and rehomogenization and the final pellet was resuspended in Tris-HCl , divided in 1 ml aliquots and stored at -70 °C.

### Binding experiment for α₂-adrenergic receptor subtypes

Membranes were thawed and re-homogenized in incubation buffer (glycylglycine 25 mM, pH 8.0). In a total volume of 500 µl, 2-10 µg protein was incubated with [³H]raulwolscine (NET-722) (New England Nuclear, USA) (1 nM final concentration) with or without competitor for 60 minutes at 25°C followed by rapid filtration over GF/B filter using a Filtermate196 harvester (Packard, Meriden, CT). Filters were rinsed extensively with ice-cold rinsing buffer (Tris-HCl 50 mM pH 7.4). Filter-bound radioactivity was determined by scintillation counting in a Topcount (Packard, Meriden, CT) and results were expressed as counts per minute (cpm). Non-specific binding was determined in the presence of 1 µM oxymetazoline for the hα_{2A} receptor and 1 µM spiroxatrine for hα_{2C} receptors.

### Example C2 : Binding experiment for the 5HT-transporter

Human platelet membranes (Oceanix Biosciences Corporation, Hanover, MD, USA) were thawed, diluted in buffer (Tris-HCl 50 mM, 120 mM NaCl and 5 mM KCl) and quickly (max 3 s) homogenised with an Ultraturrax homogenizer. In a total volume of 250 µL, 50-100 µg protein was incubated with [³H]paroxetine (NET-869) (New England Nuclear, USA) (0.5 nM final concentration) with or without competitor for 60 min at 25 °C . Incubation was stopped by rapid filtration of the incubation mixture over GF/B filters, pre-wetted with 0.1 % polyethyleneamine, using a Filtermate196 harvester (Packard, Meriden, CT). Filters were rinsed extensively with ice-cold buffer and radioactivity on the filters was counted in a Topcount liquid scintillation counter (Packard, Meriden, CT). Data were expressed as cpm. Imipramine (at 1 µM final concentration) was used to determine the non-specific binding.

### Data analysis and results

Data from assays in the presence of compound were calculated as a percentage of total binding measured in the absence of test compound. Inhibition curves, plotting percent of total binding versus the log value of the concentration of the test compound, were automatically generated, and sigmoidal inhibition curves were fitted using nonlinear regression. The pIC₅₀ values of test compounds were derived from individual curves.

All compounds according to Formula (I) produced an inhibition at least at the hα_{2C}-site (but often also at the hα_{2A}-site) of more than 50 % (pIC₅₀) at a test concentration ranging between 10⁻⁶ M and 10⁻⁹ M in a concentration-dependent manner.

Some compounds also show moderate 5-HTT activity.

For a selected number of compounds, covering most of the various embodiments of Formula (I), the results of the *in vitro* studies are given in Table 12.

**Table 12. Pharmacological data for the compounds according to the invention.**

| **Co. Nr.** | **h-**α**2A pIC₅₀** | **h-**α**2C pIC₅₀** | **h-5HTT pIC₅₀** |
|---|---|---|---|
| **7-5** | 6.3 | 7.7 | 6.0 |
| **8-1** | 6.0 | 7.6 | 5.5 |
| **8-2** | 6.5 | 7.2 | <5 |
| **7-2** | 6.1 | 7.1 | 5.5 |
| **1-3** | 6.3 | 7.0 | 5.6 |
| **7-1** | 6.5 | 7.0 | 6.1 |
| **7-4** | 5.8 | 6.9 | <5 |
| **1-4** | 6.2 | 6.9 | 6.8 |
| **6-6** | 5.4 | 6.8 | 5.8 |
| **10-11** | 6.1 | 6.7 | <5 |
| **3-5** | 6.9 | 6.7 | 5.1 |
| **11-5** | 6.1 | 6.6 | 5.5 |
| **1-1** | 6.6 | 6.6 | 6.3 |
| **3-2** | 6.7 | 6.6 | 5.2 |
| **9-3** | 5.4 | 6.5 | <5 |
| **6-5** | 5.8 | 6.5 | <5 |
| **7-10** | 5.9 | 6.5 | <5 |
| **6-9** | 5.9 | 6.5 | 5.2 |
| **1-5** | 5.9 | 6.5 | 5.3 |
| **2-2** | 6.0 | 6.5 | 6.7 |
| **10-3** | 6.1 | 6.5 | 5.0 |
| **10-10** | 6.3 | 6.5 | 5.5 |
| **3-3** | 6.5 | 6.5 | <5 |
| **10-2** | 6.6 | 6.5 | <5 |
| **3-8** | 7.1 | 6.5 | 5.0 |
| **5-5** | 5.3 | 6.4 | 5.4 |
| **7-11** | 5.8 | 6.4 | 5.7 |
| **7-3** | 5.8 | 6.4 | 5.8 |
| **6-3** | 5.9 | 6.4 | <5 |
| **9-6** | 5.9 | 6.4 | 5.2 |
| **6-2** | 5.9 | 6.4 | 5.4 |
| **6-8** | 6.0 | 6.4 | 5.3 |
| **7-15** | 6.0 | 6.4 | 5.5 |
| **1-7** | 6.0 | 6.4 | 6.0 |
| **6-7** | 6.1 | 6.4 | 5.3 |
| **7-14** | 6.3 | 6.4 | 5.4 |
| **7-12** | 5.7 | 6.3 | 5.6 |
| **7-13** | 5.8 | 6.3 | 6.0 |
| **10-12** | 5.9 | 6.3 | <5 |
| **9-9** | 5.9 | 6.3 | 5.2 |
| **9-1** | 6.0 | 6.3 | 5.0 |
| **10-6** | 6.0 | 6.3 | 5.3 |
| **7-8** | 6.1 | 6.3 | 5.3 |
| **1-6** | 6.1 | 6.3 | 6.4 |
| **4-1** | 6.3 | 6.3 | 5.4 |
| **6-4** | 5.5 | 6.2 | <5 |
| **9-4** | 5.5 | 6.2 | <5 |
| **10-4** | 5.6 | 6.2 | <5 |
| **9-10** | 5.6 | 6.2 | <5 |
| **1-9** | 5.7 | 6.2 | 6.5 |
| **9-8** | 5.9 | 6.2 | 5.3 |
| **2-1** | 5.9 | 6.2 | 5.8 |
| **5-2** | 6.0 | 6.2 | <5 |
| **9-7** | 6.0 | 6.2 | < 5 |
| **5-1** | 6.0 | 6.2 | 5.1 |
| **10-1** | 6.1 | 6.2 | <5 |
| **11-3** | 6.1 | 6.2 | <5 |
| **10-8** | 6.1 | 6.2 | 5.3 |
| **9-2** | 6.1 | 6.2 | 5.6 |
| **11-7** | 6.2 | 6.2 | 5.8 |
| **10-5** | 6.5 | 6.2 | <5 |
| **11-1** | 6.6 | 6.2 | 5.7 |
| **4-3** | 6.7 | 6.2 | <5 |
| **3-1** | 7.1 | 6.2 | 5.3 |
| **7-6** | 5.4 | 6.1 | <5 |
| **1-10** | 5.5 | 6.1 | <5 |
| **9-5** | 5.6 | 6.1 | <5 |
| **7-9** | 5.8 | 6.1 | <5 |
| **7-7** | 5.8 | 6.1 | <5 |
| **11-4** | 5.8 | 6.1 | <5 |
| **3-9** | 5.8 | 6.1 | 6.1 |
| **1-8** | 6.0 | 6.1 | 6.2 |
| **11-2** | 6.1 | 6.1 | <5 |
| **3-6** | 6.1 | 6.1 | 5.3 |
| **11-6** | 6.2 | 6.1 | 5.8 |
| **1-11** | 6.2 | 6.1 | 6.3 |
| **4-2** | 6.3 | 6.1 | <5 |
| **3-4** | 6.7 | 6.1 | 5.0 |
| **10-9** | 6.9 | 6.1 | 5.0 |
| **6-1** | 5.4 | 6.0 | <5 |
| **5-4** | 5.5 | 6.0 | <5 |
| **1-2** | 5.6 | 6.0 | 5.5 |
| **10-7** | 5.7 | 6.0 | <5 |
| **5-3** | 6.0 | 6.0 | <5 |
| **3-10** | 6.3 | 6.0 | 5.2 |
| **3-7** | 6.7 | 6.0 | 5.3 |

### D. Composition examples

"Active ingredient" (a.i.) as used throughout these examples relates to a compound of formula (i), the pharmaceutically acceptable acid or base addition salts thereof, the stereochemically isomeric forms thereof, the *N*-oxide form thereof, a quaternary ammonium salt thereof and prodrugs thereof.

### Example D.1 : oral drops

500 Grams of the a.i. is dissolved in 0.5 l of 2-hydroxypropanoic acid and 1.5 l of the polyethylene glycol at 60-80 °c. After cooling to 30-40 °C there are added 35 l of polyethylene glycol and the mixture is stirred well. Then there is added a solution of 1750 grams of sodium saccharin in 2.5 l of purified water and while stirring there are added 2.5 l of cocoa flavor and polyethylene glycol q.s. to a volume of 50 I, providing an oral drop solution comprising 10 mg/ml of a.i. The resulting solution is filled into suitable containers.

### Example D.2 : oral solution

9 Grams of methyl 4-hydroxybenzoate and 1 gram of propyl 4-hydroxybenzoate are dissolved in 4 l of boiling purified water. In 3 l of this solution are dissolved first 10 grams of 2,3-dihydroxybutanedioic acid and thereafter 20 grams of the a.i. The latter solution is combined with the remaining part of the former solution and 12 l 1,2,3-propanetriol and 3 l of sorbitol 70 % solution are added thereto. 40 Grams of sodium saccharin are dissolved in 0.5 l of water and 2 ml of raspberry and 2 ml of gooseberry essence are added. The latter solution is combined with the former, water is added q.s. to a volume of 20 l providing an oral solution comprising 5 mg of the active ingredient per teaspoonful (5 ml). The resulting solution is filled in suitable containers.

### Example D.3 : film-coated tablets

### Preparation of tablet core

A mixture of 100 grams of the a.i., 570 grams lactose and 200 grams starch is mixed well and thereafter humidified with a solution of 5 grams sodium dodecyl sulfate and 10 grams polyvinylpyrrolidone in about 200 ml of water. The wet powder mixture is sieved, dried and sieved again. Then there is added 100 grams microcrystalline cellulose and 15 grams hydrogenated vegetable oil. The whole is mixed well and compressed into tablets, giving 10,000 tablets, each containing 10 mg of the active ingredient.

### Coating

To a solution of 10 grams methyl cellulose in 75 ml of denaturated ethanol there is added a solution of 5 grams of ethyl cellulose in 150 ml of dichloromethane. Then there are added 75 ml of dichloromethane and 2.5 ml 1,2,3-propanetriol. 10 grams of polyethylene glycol is molten and dissolved in 75 ml of dichloromethane. The latter solution is added to the former and then there are added 2.5 grams of magnesium octadecanoate, 5 grams of polyvinylpyrrolidone and 30 ml of concentrated color suspension and the whole is homogenated. The tablet cores are coated with the thus obtained mixture in a coating apparatus.

### Example D.4 : injectable solution

1.8 grams methyl 4-hydroxybenzoate and 0.2 grams propyl 4-hydroxybenzoate are dissolved in about 0.5 l of boiling water for injection. After cooling to about 50 °C there are added while stirring 4 grams lactic acid, 0.05 grams propylene glycol and 4 grams of the a.i.. The solution is cooled to room temperature and supplemented with water for injection q.s. ad 1 I, giving a solution comprising 4 mg/ml of a.i.. The solution is sterilized by filtration and filled in sterile containers.

### E. Physico-chemical data

### General procedure

The HPLC gradient was supplied by a HP 1100 from Agilent Technologies comprising a pump (quaternary or binary) with degasser, an autosampler, a column oven (set at 40 °C), a diode-array detector (DAD) and a column as specified in the respective methods below. Flow from the column was split to a MS detector. The MS detector was configured with an electrospray ionization source. Nitrogen was used as the nebulizer gas. The source temperature was maintained at 140 °C. Data acquisition was performed with MassLynx-Openlynx software.

### E.1 LCMS - PROCEDURE 1

In addition to the general procedure: Reversed phase HPLC was carried out on an XDB-C18 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. High-resolution mass spectra (Time of Flight, TOF) were acquired by scanning from 100 to 750 in 1.0 second using a dwell time of 1.0 second. The capillary needle voltage was 2.5 kV for positive ionization mode and 2.9 kV for negative ionization mode. The cone voltage was 20 V for both positive and negative ionization modes. Leucine-enkephaline was the standard substance used for the lock mass calibration.

### E.2 LCMS - PROCEDURE 2

In addition to the general procedure: Reversed phase HPLC was carried out on an XDB-C18 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. High-resolution mass spectra (Time of Flight, TOF) were acquired only in positive ionization mode by scanning from 100 to 750 in 0.5 seconds using a dwell time of 0.1 seconds. The capillary needle voltage was 2.5 kV and the cone voltage was 20 V. Leucine-enkephaline was the standard substance used for the lock mass calibration.

**Table 13: Analytical data**

| **Co. Nr.** | **Rₜ** | **(MH)⁺** | **Procedure** | **Physico-chemical data** |
|---|---|---|---|---|
| **1-1** | 4.09 | 480 | 1 | |
| **1-2** | 4.66 | 430 | 1 | |
| **1-3** | 5.48 | 472 | 1 | |
| **1-4** | 5.56 | 548 | 1 | Trifluoroacetate-salt . 2C₂HF₃O₂ |
| **1-5** | 4.73 | 433 | 1 | |
| **1-6** | 6.16 | 529 | 1 | |
| **1-7** | 6.13 | 529 | 1 | |
| **1-8** | 6.16 | 529 | 1 | |
| **1-9** | 5.66 | 459 | 1 | |
| **1-10** | 4.75 | 452 | 1 | |
| **2-1** | 5.85 | 504 | 1 | |
| **2-2** | 5.76 | 534 | 1 | |
| **3-1** | 4.61 | 466 | 1 | Trifluoroacetate-salt . 2C₂HF₃O₂ |
| **3-2** | 6.05 | 566 | 1 | |
| **3-3** | 4.67 | 347 | 1 | |
| **3-4** | 5.50 | 387 | 1 | |
| **3-5** | 5.99 | 437 | 1 | |
| **3-6** | 6.18 | 463 | 1 | |
| **3-7** | 6.30 | 487 | 1 | |
| **3-8** | 5.68 | 427 | 1 | |
| **3-9** | 5.76 | 534 | 1 | |
| **3-10** | 5.68 | 535 | 1 | |
| **4-2** | 3.98 | 375 | 1 | Trifluoroacetate-salt . C₂HF₃O₂ |
| **4-3** | 5.73 | 475 | 1 | |
| **5-1** | 2.69 | 404 | 1 | |
| **5-2** | 2.75 | 404 | 1 | Trifluoroacetate-salt . 2C₂HF₃O₂ |
| **5-4** | 3.89 | 396 | 1 | |
| **5-5** | 4.38 | 472 | 1 | Trifluoroacetate-salt . 2C₂HF₃O₂ |
| **6-1** | 1.94 | 356 | 1 | |
| **6-2** | 2.73 | 418 | 1 | |
| **6-3** | 2.62 | 418 | 1 | Trifluoroacetate-salt . 2C₂HF₃O₂ |
| **6-4** | 4.35 | 456 | 1 | |
| **6-5** | 3.95 | 410 | 1 | |
| **6-6** | 4.25 | 486 | 1 | |
| **6-7** | 4.99 | 467 | 1 | |
| **6-8** | 5.20 | 467 | 1 | |
| **6-9** | 5.06 | 467 | 1 | |
| **7-1** | 2.87 | 432 | 1 | |
| **7-2** | 4.45 | 470 | 1 | |
| **7-3** | 4.91 | 532 | 1 | |
| **7-4** | 3.88 | 424 | 1 | |
| **7-5** | 4.23 | 500 | 1 | Trifluoroacetate-salt . 2C₂HF₃O₂ |
| **7-6** | 3.05 | 385 | 1 | |
| **7-7** | 2.48 | 313 | 1 | |
| **7-8** | 3.71 | 355 | 1 | |
| **7-9** | 2.74 | 368 | 1 | |
| **7-10** | 3.95 | 411 | 1 | |
| **7-11** | 4.97 | 481 | 1 | |
| **7-12** | 4.96 | 481 | 1 | |
| **7-13** | 5.01 | 481 | 1 | |
| **7-14** | 3.95 | 431 | 1 | |
| **7-15** | 3.76 | 387 | 1 | |
| **8-1** | 4.37 | 500 | 2 | Trifluoroacetate-salt . C₂HF₃O₂ |
| **8-2** | 4.56 | 445 | 2 | |
| **9-1** | 3.67 | 432 | 1 | |
| **9-2** | 4.33 | 494 | 1 | |
| **9-3** | 5.65 | 532 | 1 | |
| **9-4** | 5.93 | 594 | 1 | |
| **9-5** | 5.57 | 486 | 1 | |
| **9-6** | 5.53 | 562 | 1 | |
| **9-7** | 6.12 | 543 | 1 | |
| **9-8** | 6.13 | 543 | 1 | |
| **9-9** | 6.12 | 543 | 1 | |
| **9-10** | 5.40 | 493 | 1 | |
| **10-1** | 3.18 | 404 | 1 | Trifluoroacetate-salt . 2C₂HF₃O₂ |
| **10-2** | 5.30 | 490 | 1 | |
| **10-3** | 5.30 | 504 | 1 | |
| **10-4** | 4.28 | 325 | 1 | |
| **10-5** | 5.42 | 387 | 1 | |
| **10-6** | 5.45 | 401 | 1 | |
| **10-7** | 4.67 | 403 | 1 | |
| **10-8** | 5.96 | 451 | 1 | |
| **10-9** | 5.64 | 411 | 1 | |
| **10-10** | 5.65 | 425 | 1 | |
| **10-11** | 5.51 | 415 | 1 | |
| **10-12** | 4.75 | 473 | 1 | |
| **11-1** | 4.01 | 510 | 1 | Trifluoroacetate-salt . 2C₂HF₃O₂ |
| **11-2** | 4.00 | 476 | 1 | |
| **11-3** | 5.39 | 548 | 1 | |
| | | | | |
| **11-5** | 5.13 | 578 | 1 | Trifluoroacetate-salt . C₂HF₃O₂ |
| **11-6** | 5.91 | 559 | 1 | |
| **11-7** | 5.92 | 559 | 1 | |

## Claims

1. Compound according to the general Formula (I) 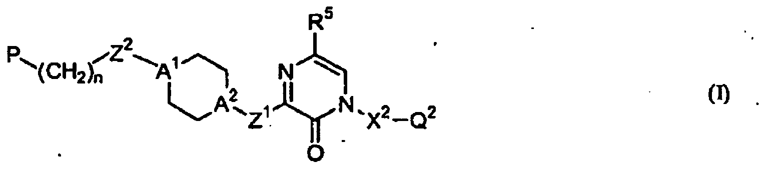 a pharmaceutically acceptable acid or base addition salt thereof, an *N*-oxide form thereof or a quaternary ammonium salt thereof, wherein :
A¹, A² are each, independently from each other, a nitrogen or a carbon-atom; with the provision that A¹ and A² are not simultaneously a carbon atom;
Z¹, **Z²** are each, independently from each other, a covalent bond or N-R⁴ ; wherein R⁴ is selected from the group of hydrogen, (C₁-₃)alkyl, aryl and aryl(C₁₋₃)alkyl;
n is an integer equal to zero, 1, 2 or 3 ;
R⁵ is selected from the group of hydrogen and halo ;
P is a radical selected from the group of phenyl, biphenyl, 1,1-diphenylmethyl and benzyloxyphenyl ;
X² is a covalent bond, a saturated or an unsaturated (C₁₋₈)-hydrocarbon radical, wherein one or more bivalent -CH₂-units may optionally be replaced by a respective bivalent phenyl-unit ; and wherein one or more hydrogen atoms may be replaced by a radical selected from the group of oxo ; (C₁₋₃)alkyloxy ; halo ; cyano ; nitro ; formyl ; hydroxy ; amino ; trifluoromethyl ; mono- and di((C₁₋₃)alkyl)amino ; carboxyl ; and thio ;
Q² is a radical selected from the group of hydrogen ; -NR¹R² ; Pir ; -OR^{3a} ; SR^{3b} ; SO₂R^{3c} ; aryl ; and Het; wherein two radicals -OR^{3a} may be taken together to form a bivalent radical -O-(CH₂)₅-O- wherein s is an integer equal to 1, 2 or 3 ;
R¹ and R² are each, independently from each other, a radical selected from the group of hydrogen ; alkyl ; alkenyl alkynyl ; aryl ; arylalkyl ; diarylalkyl ; alkylcarbonyl ; alkylcarbonylalkyl ; alkenylcarbonyl ; alkyloxy ; alkyloxyalkyl ; alkyloxycarbonyl ; alkyloxyalkylcarbonyl; alkyloxycarbonylalkyl ; alkyloxycarbonylalkylcarbonyl; alkylsulfonyl ; arylsulfonyl ; arylalkylsulfonyl ; arylalkenylsulfonyl ; Het-sulfonyl ; arylcarbonyl ; aryloxyalkyl ; arylalkylcarbonyl ; Het ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein R^{a} and R^{b} are each independently selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl ;
Pir is a radical containing at least one N, by which it is attached to the X²-radical, selected from the group of pyrrolidinyl ; imidazolidinyl ; pyrazolidinyl; piperidinyl ; piperazinyl ; pyrrolyl ; pyrrolinyl ; imidazolinyl ; pyrrazolinyl ; pyrrolyl ; imidazolyl ; pyrazolyl ; triazolyl ; azepyl ; diazepyl; morpholinyl ; thiomorpholinyl ; indolyl ; isoindolyl; indolinyl ; indazolyl ; benzimidazolyl ; and 1,2,3,4-tetrahydro-isoquinolinyl ; wherein each Pir-radical is optionally substituted by 1, 2 or 3 radicals selected from the group of hydroxy ; halo ; oxo ; (C₁₋₃)alkyl ; (C₁₋₃)alkenyl; (C₁₋₃)alkyloxycarbonyl ; Het-carbonyl ; (C₁₋₃)alkylamino ; trifluoromethyl ; phenyl(C₀₋₃)alkyl ; pyrimidinyl ; pyrrolidinyl ; and pyridinyloxy ;
R^{3a} is a radical selected from the group of hydrogen ; alkyl ; trihaloalkyl ; arylalkyl ; alkyloxyalkyl ; Het ; and Het-alkyl ;
R^{3b}, R^{3c} are each, independently from each other, a radical selected from the group of hydrogen ; alkyl ; trihaloalkyl ; aryl ; arylalkyl ; alkyloxyalkyl ; Het ; and Het-alkyl ;
Het is a heterocyclic radical selected from the group of pyrrolidinyl ; imidazolidinyl ; pyrazolidinyl; piperidinyl ; piperazinyl ; pyrrolyl ; pyrrolinyl ; imidazolinyl ; pyrrazolinyl ; pyrrolyl ; imidazolyl ; pyrazolyl ; triazolyl ; pyridinyl ; pyridazinyl; pyrimidinyl pyrazinyl ; triazinyl ; azepyl ; diazepyl ; morpholinyl ; thiomorpholinyl ; indolyl ; isoindolyl ; indolinyl ; indazolyl ; benzimidazolyl ; 1,2,3,4-tetrahydro-isoquinolinyl ; furyl ; tetrahydropyranyl ; thienyl ; oxazolyl ; isoxazolyl ; thiazolyl ; thiadiazolyl ; isothiazolyl ; dioxolyl ; dithianyl ; tetrahydrofuryl ; tetrahydropyranyl ; oxadiazolyl ; quinolinyl ; isoquinolinyl ; quinoxalinyl ; benzoxazolyl ; benzisoxazolyl; benzothiazolyl; benzisothiazolyl ; benzofuranyl; benzothienyl ; benzopiperidinyl ; benzomorpholinyl ; chromenyl ; and imidazo[1,2-*a*]pyridinyl ; wherein each Het-radical is optionally substituted by one or more radicals selected from the group of halo ; oxo ; (C₁₋₃)alkyl ; phenyl, optionally substituted with (C₁₋₃)alkyloxy ; (C₁₋₃)alkylcarbonyl ; (C₁₋₃)alkenylthio ; imidazolyl-(C₁₋₃)alkyl ; aryl(C₁₋₃)alkyl and (C₁₋₃)alkyloxycarbonyl ;
aryl is naphthyl or phenyl, each optionally substituted with 1, 2 or 3 substituents, each independently from each other, selected from the group of oxo ; (C₁₋₃)alkyl; (C₁₋₃)alkyloxy ; halo ; cyano ; nitro ; formyl ; ethanoyl : hydroxy ; amino; trifluoromethyl ; mono- and di((C₁₋₃)alkyl)amino ; mono- and di((C₁₋₃)alkylcarbony)amino ; carboxyl; morpholinyl ; and thio ;
alkyl is, unless otherwise indicated, a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms; wherein each radical is optionally substituted on one or more carbon atoms with one or more radicals selected from the group of oxo ; (C₁₋₃)alkyloxy, halo ; cyano ; nitro ; formyl ; hydroxy ; amino ; carboxyl ; and thio ;
alkenyl is an alkyl radical as defined above, further having one or more double bonds;
alkynyl is an alkyl radical as defined above, further having one or more triple bonds;
arylalkyl is an alkyl radical as defined above, further having one CH₃-group replaced by phenyl ; and
diarylalkyl is an alkyl radical as defined above, further having two CH₃-groups replaced by phenyl.

2. Compound according to claim 1, wherein the moiety 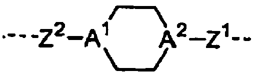 is a bivalent radical of formula (II-a), (II-b), (II-c) and (II-d), as shown below.

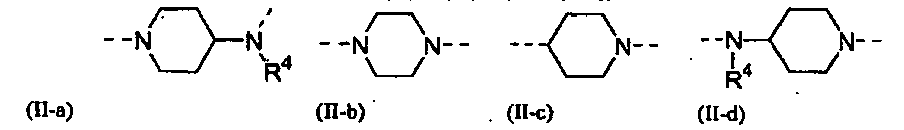

3. Compound according to claim 2, **characterized in that** R⁴ is hydrogen or p-aminomethylbenzyl.

4. Compound according to any one of claims 1 to 3, **characterized in that** n is 1, 2 or 3.

5. Compound according to any one of claims 1 to 4. **characterized in that** R⁵ is hydrogen.

6. Compound according to any one of claims 1 to 5, **characterized in that** P is phenyl.

7. Compound according to any one of claims 1 to 6, **characterized in that** X² is selected from the group of a covalent bond, a C₁-hydrocarbon radical, a C₂-hydrocarbon radical, or a C₃-hydrocarbon radical.

8. Compound according to any one of claims 1 to 7, **characterized in that** one bivalent -CH₂-unit of the hydrocarbon radical X² is replaced by a bivalent phenyl-unit; or wherein two hydrogen atoms of the hydrocarbon radical X² are replaced by an oxo-radical.

9. Compound according to any one of claims 1 to 8, **characterized in that** X² is selected from the group of a covalent bond and any one of the radicals (aa), (ab), (ac), (ag), (am), (an), (aq). (as) and (be) as defined below:
| | | | |
|---|---|---|---|
| -CH₂- | (aa) | 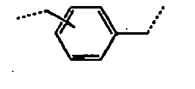 | (be) |
| -CH₂CH₂- | (ab) | | |
| -CH₂CH₂CH₂- | (ac) | | |
| | | | |
| -CH₂CH=CH- | (ag) | | |
| | | | |
| -C(=O)CH₂- | (am) | | |
| -C(=O)CH₂CH₂- | (an) | | |
| -CH₂C(=O)CH₂- | (aq) | | |
| -CH₂C(=O)C(CH₃)₂CH₂- | (as) | | |

10. Compound according to any one of claims 1 to 9, **characterized in that** Q² is a radical selected from the group of hydrogen ; -NR¹R² ; Pir ; -OR^{3a} ; SR^{3b} ; aryl ; and Het.

11. Compound according to any one of claims 1 to 10, **characterized in that** R¹ and R² are each, independently from each other, a radical selected from the group of hydrogen ; alkyl and alkyloxycarbonyl.

12. Compound according to any one of claims 1 to 10, **characterized in that** Pir is a radical containing at least one N, by which it is attached to the X²-radical, selected from the group of piperidinyl and isoindolyl ; wherein each Pir-radical is optionally substituted by 2 oxo-radicals.

13. Compound according to any one of claims 1 to 10, **characterized in that** R^{3a} and R^{3b} are each, independently from each other, an alkyl-radical.

14. Compound according to any one of claims 1 to 10, **characterized in that** Het is a heterocyclic radical selected from the group of pyridinyl ; furyl; tetrahydropyranyl; thienyl ; oxadiazolyl ; and quinolinyl ; wherein each Het-radical is optionally substituted by one or more radicals selected from the group of halo and phenyl, optionally substituted with (C₁₋₃)alkyloxy.

15. Compound according to any one of claims 1 to 10, **characterized in that** aryl is naphthyl or phenyl, each optionally substituted with a substituent, selected from the group of (C₁₋₃)alkyl and halo.

16. Compound according to claim 1, **characterized in that :**
A¹, A² are each, independently from each other, a nitrogen or a carbon-atom; with the provision that A¹ and A² are not simultaneously a carbon atom;
Z¹, Z² are each, independently from each other, a covalent bond or N-R⁴ ; wherein R⁴ is selected from the group of hydrogen and aryl-(C₁₋₃)alkyl ;
n is an integer equal to zero, 1, 2 or 3 ;
R⁵ is hydrogen ;
P is a radical selected from the group of phenyl, biphenyl, 1,1-diphenylmethyl and benzyloxyphenyl ;
X² is a bond, a saturated or an unsaturated (C₁₋₈)-hydrocarbon radical, wherein one or more bivalent -CH₂-units may optionally be replaced by a respective bivalent phenyl-unit ; and/or wherein one or more hydrogen atoms may be replaced by an oxo-radical ;
Q² is a radical selected from the group of hydrogen ; -NR¹R² ; Pir ; -OR^{3a} ; SR^{3b} ; aryl ; and Het;
R¹ and R² are each, independently from each other, a radical selected from the group of hydrogen; alkyl ; and alkyloxycarbonyl ;
Pir is a radical containing at least one N, by which it is attached to the X²-radical, selected from the group of piperidinyl ; isoindolyl; wherein each Pir-radical is optionally substituted by 2 oxo-radicals ;
R^{3a}, R^{3b}, R^{3c} are each, independently from each other, an alkyl-radical ;
Het is a heterocyclic radical selected from the group of pyridinyl ; furyl ; tetra-hydropyranyl ; thienyl ; oxadiazolyl ; and quinolinyl ; wherein each Het-radical is optionally substituted by one or more radicals selected from the group of halo ; and phenyl, optionally substituted with (C₁₋₃)alkyloxy ; and
aryl is naphthyl or phenyl, each optionally substituted with a substituent, each independently from each other, selected from the group of (C₁₋₃)alkyl and halo.

17. Compound according to any one of claims 1 to 16 for use as a medicine.

18. Pharmaceutical composition comprising a pharmaceutically acceptable carrier or diluent and, as active ingredient, a therapeutically effective amount of a compound according to any one of claims 1 to 16.

19. Pharmaceutical composition according to claim 18, **characterized in that** is comprises further one or more other compounds selected from the group of antidepressants, anxiolytics and antipsychotics.

20. Pharmaceutical composition according to any of claims 18 and 19, **characterized in that** it is in a form suitable to be orally administered.

21. Process for the preparation of a pharmaceutical composition as claimed in any one of claims 18 to 20, **characterized in that** a pharmaceutically acceptable carrier is intimately mixed with a therapeutically effective amount of a compound as claimed in any one of claims 1 to 16.

22. Process for the preparation of a pharmaceutical composition as claimed in any one of claims 18 to 20, **characterized in that** a pharmaceutically acceptable carrier is intimately mixed with a therapeutically effective amount of a compound as claimed in any one of claims 1 to 16 and one or more other compounds selected from the group of antidepressants, anxiolytics and antipsychotics.

23. Use of a compound according to any one of claims 1 to 16 for the preparation of a medicament for the prevention and/or treatment of diseases where antagonism of the α₂-adrenergic receptor, in particular antagonism of the α_{2C}-adrenergic receptor is of therapeutic use.

24. Use of a compound according to any one of claims 1 to 16 for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders and sexual dysfunction.

25. Use of a compound according to any one of claims 1 to 16 in combination with one or more other compounds selected from the group of antidepressants, anxiolytics and antipsychotics for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders and sexual dysfunction.

## Patentansprüche

1. Verbindungen der allgemeinen Formel (I) 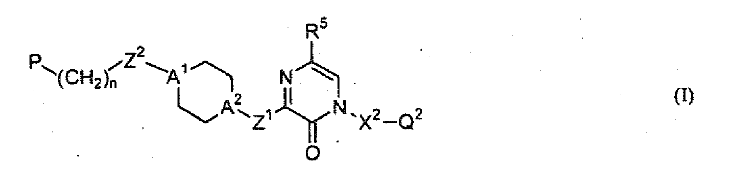 deren pharmazeutisch unbedenkliche Säure- oder Basenadditionssalze, deren N-Oxidformen und deren quaternäre Ammoniumsalze, wobei:
A¹, A² jeweils unabhängig voneinander für ein Stickstoff- oder ein Kohlenstoffatom stehen; mit der Maßgabe, daß A¹ und A² nicht beide für ein Kohlenstoffatom stehen;
Z¹, Z² jeweils unabhängig voneinander für eine kovalente Bindung oder N-R⁴ stehen; wobei R⁴ aus der aus Wasserstoff, (C₁₋₃)-Alkyl, Aryl und Aryl-(C₁₋₃)-alkyl bestehenden Gruppe ausgewählt ist;
n für eine ganze Zahl gleich Null, 1, 2 oder 3 steht;
R⁵ aus der aus Wasserstoff und Halogen bestehenden Gruppe ausgewählt ist;
P für einen aus der aus Phenyl, Biphenyl, 1, 1-Diphenylmethyl und Benzyloxyphenyl bestehenden Gruppe ausgewählten Rest steht;
X² für eine kovalente Bindung, einen gesättigten oder einen ungesättigten (C₁₋₈)-Kohlenwasserstoffrest, wobei eine oder mehrere zweiwertige -CH₂-Einheiten gegebenenfalls durch eine entsprechende zweiwertige Phenyleinheit ersetzt sein können; und wobei ein oder mehrere Wasserstoffatome durch einen Rest ausgewählt aus der aus Oxo, (C₁₋₃)-Alkyloxy, Halogen, Cyano, Nitro, Formyl, Hydroxy, Amino, Trifluormethyl, Mono- und Di- ((C₁₋₃) -alkyl) amino, Carboxyl und Thio bestehenden Gruppe ersetzt sein können, steht;
Q² für einen aus der aus Wasserstoff, -NR¹R², Pir, -OR^{3a}, SR^{3b}, SO₂R^{3c}, Aryl und Het bestehenden Gruppe ausgewählten Rest steht, wobei zwei Reste -OR^{3a} zusammen einen zweiwertigen Rest -O-(CH₂)ₛ-O- bilden können, wobei s für eine ganze Zahl gleich 1, 2 oder 3 steht;
R¹ und R² jeweils unabhängig voneinander für einen aus der aus Wasserstoff, Alkyl, Alkenyl, Alkynyl, Aryl, Arylalkyl, Diarylalkyl, Alkylcarbonyl, Alkylcarbonylalkyl, Alkenylcarbonyl, Alkyloxy, Alkyloxyalkyl, Alkyloxycarbonyl, Alkyloxyalkylcarbonyl, Alkyloxycarbonylalkyl, Alkyloxycarbonylalkylcarbonyl, Alkylsulfonyl, Arylsulfonyl, Arylalkylsulfonyl, Arylalkenylsulfonyl, Het-Sulfonyl, Arylcarbonyl, Aryloxyalkyl, Arylalkylcarbonyl, Het, Het-Alkyl, Het-Alkylcarbonyl, Het-Carbonyl, Het-Carbonylalkyl, Alkyl-NR^{a}R^{b}, Carbonyl-NR^{a}R^{b}, Carbonylalkyl-NR^{a}R^{b}, Alkylcarbonyl-NR^{a}R^{b} und Alkylcarbonylalkyl-NR^{a}R^{b} bestehenden Gruppe ausgewählten Rest stehen; wobei R^{a} und R^{b} jeweils unabhängig aus der aus Wasserstoff, Alkyl, Alkylcarbonyl, Alkyloxyalkyl, Alkyloxycarbonylalkyl, Aryl, Arylalkyl, Het und Alkyl-NR^{c}R^{d} bestehenden Gruppe ausgewählt sind, wobei R^{c} und R^{d} jeweils unabhängig voneinander für Wasserstoff oder Alkyl stehen;
Pir für einen wenigstens einen Stickstoff enthaltenden Rest steht, der über den Stickstoff an den X²-Rest gebunden ist, ausgewählt aus der aus Pyrrolidinyl, Imidazolidinyl, Pyrazolidinyl, Piperidinyl, Piperazinyl, Pyrrolyl, Pyrrolinyl, Imidazolinyl, Pyrrazolinyl, Pyrrolyl, Imidazolyl, Pyrazolyl, Triazolyl, Azepyl, Diazepyl, Morpholinyl, Thiomorpholinyl, Indolyl, Isoindolyl, Indolinyl, Indazolyl, Benzimidazolyl und 1,2,3,4-Tetrahydroisochinolinyl bestehenden Gruppe, wobei die Pir-Reste jeweils gegebenenfalls durch 1, 2 oder 3 Reste ausgewählt aus der aus Hydroxy, Halogen, Oxo, (C₁₋₃)-Alkyl, (C₁₋₃)-Alkenyl, (C₁₋₃)-Alkyloxycarbonyl, Het-Carbonyl, (C₁₋₃)-Alkylamino, Trifluormethyl, Phenyl-(C₀₋₃)-alkyl, Pyrimidinyl, Pyrrolidinyl und Pyridinyloxy bestehenden Gruppe substituiert sind;
R^{3a} für einen aus der aus Wasserstoff, Alkyl, Trihalogenalkyl, Arylalkyl, Alkyloxyalkyl, Het und Het-Alkyl bestehenden Gruppe ausgewählten Rest steht;
R^{3b}, R^{3c} jeweils unabhängig voneinander für einen aus der aus Wasserstoff, Alkyl, Trihalogenalkyl, Aryl, Arylalkyl, Alkyloxyalkyl, Het und Het-Alkyl bestehenden Gruppe ausgewählten Rest stehen;
Het für einen aus der aus Pyrrolidinyl, Imidazolidinyl, Pyrazolidinyl, Piperidinyl, Piperazinyl, Pyrrolyl, Pyrrolinyl, Imidazolinyl, Pyrrazolinyl, Pyrrolyl, Imidazolyl, Pyrazolyl, Triazolyl, Pyridinyl, Pyridazinyl, Pyrimidinyl, Pyrazinyl, Triazinyl, Azepyl, Diazepyl, Morpholinyl, Thiomorpholinyl, Indolyl, Isoindolyl, Indolinyl, Indazolyl, Benzimidazolyl, 1,2,3,4-Tetrahydroisochinolinyl, Furyl, Tetrahydropyranyl, Thienyl, Oxazolyl, Isoxazolyl, Thiazolyl, Thiadiazolyl, Isothiazolyl, Dioxolyl, Dithianyl, Tetrahydrofuryl, Tetrahydropyranyl, Oxadiazolyl, Chinolinyl, Isochinolinyl, Chinoxalinyl, Benzoxazolyl, Benzisoxazolyl, Benzothiazolyl, Benzisothiazolyl, Benzofuranyl, Benzothienyl, Benzopiperidinyl, Benzomorpholinyl, Chromenyl und Imidazo[1,2-α]pyridinyl bestehenden Gruppe ausgewählten heterocyclischen Rest steht; wobei die Het-Reste jeweils gegebenenfalls durch einen oder mehrere aus der aus Halogen, Oxo, (C₁₋₃)-Alkyl, gegebenenfalls durch (C₁₋₃)-Alkyloxy substituiertes Phenyl, (C₁₋₃)-Alkylcarbonyl, (C₁₋₃)-Alkenylthio, Imidazolyl-(C₁₋₃)-alkyl, Aryl-(C₁₋₃)-alkyl und (C₁₋₃)-Alkyloxycarbonyl bestehenden Gruppe ausgewählte Reste substituiert sind;
Aryl für Naphthyl oder Phenyl steht, die jeweils gegebenenfalls durch 1, 2 oder 3 Substituenten, die jeweils unabhängig voneinander aus der aus Oxo, (C₁₋₃)-Alkyl, (C₁₋₃)-Alkyloxy, Halogen, Cyano, Nitro, Formyl, Ethanoyl, Hydroxy, Amino, Trifluormethyl, Mono- und Di-((C₁₋₃)-alkyl)amino, Mono- und Di-((C₁₋₃)-alkylcarbonyl)amino, Carboxyl, Morpholinyl und Thio bestehenden Gruppe ausgewählt sind, substituiert sind;
Alkyl, wenn nicht anders angegeben, für einen geradkettigen oder verzweigten gesättigten Kohlenwasserstoffreste mit 1 bis 8 Kohlenstoffatomen oder einen cyclischen gesättigten Kohlenwasserstoffrest mit 3 bis 7 Kohlenstoffatomen oder einen cyclischen gesättigten Kohlenwasserstoffrest mit 3 bis 7 Kohlenstoffatomen, der an einen geradkettigen oder verzweigten gesättigten Kohlenwasserstoffrest mit 1 bis 8 Kohlenstoffatomen gebunden ist, steht; wobei die Reste jeweils gegebenenfalls an einem oder mehreren Kohlenstoffatomen durch einen oder mehrere aus der aus Oxo, (C₁₋₃)-Alkyloxy, Halogen, Cyano, Nitro, Formyl, Hydroxy, Amino, Carboxyl und Thio bestehenden Gruppe ausgewählte Reste substituiert sind;
Alkenyl für einen wie oben definierten Alkylrest steht, der weiterhin eine oder mehrere Doppelbindungen aufweist;
Alkynyl für einen wie oben definierten Alkylrest steht, der weiterhin eine oder mehrere Dreifachbindungen aufweist;
Arylalkyl für einen wie oben definierten Alkylrest steht, bei dem weiterhin eine CH₃-Gruppe durch Phenyl ersetzt ist; und
Diarylalkyl für einen wie oben definierten Alkylrest steht, bei dem weiterhin zwei CH₃-Gruppen durch Phenyl ersetzt sind.

2. Verbindungen nach Anspruch 1, wobei die Einheit 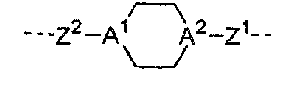 für einen zweiwertigen Rest der Formel (II-a), (II-b), (II-c) oder (II-d), wie unten gezeigt, steht.

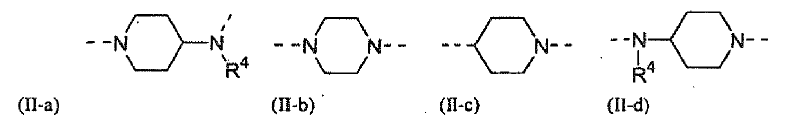

3. Verbindungen nach Anspruch 2, **dadurch gekennzeichnet, daß** R⁴ für Wasserstoff oder p-Aminomethylbenzyl steht.

4. Verbindungen nach einem der Ansprüche 1 bis 3, **dadurch gekennzeichnet, daß** n für 1, 2 oder 3 steht.

5. Verbindungen nach einem der Ansprüche 1 bis 4, **dadurch gekennzeichnet, daß** R⁵ für Wasserstoff steht.

6. Verbindungen nach einem der Ansprüche 1 bis 5, **dadurch gekennzeichnet, daß** P für Phenyl steht.

7. Verbindungen nach einem der Ansprüche 1 bis 6, **dadurch gekennzeichnet, daß** X² aus der aus einer kovalenten Bindung, einem C₁-Kohlenwasserstoffrest, einem C₂-Kohlenwasserstoffrest und einem C₃-Kohlenwasserstoffrest bestehenden Gruppe ausgewählt ist.

8. Verbindungen nach einem der Ansprüche 1 bis 7, **dadurch gekennzeichnet, daß** eine zweiwertige -CH₂-Einheit des Kohlenwasserstoffrests X² durch eine zweiwertige Phenyleinheit ersetzt ist; oder wobei zwei Wasserstoffatome des Kohlenwasserstoffrests X² durch einen Oxorest ersetzt sind.

9. Verbindungen nach einem der Ansprüche 1 bis 8, **dadurch gekennzeichnet, daß** X² aus der aus einer kovalenten Bindung und einem der Reste (aa), (ab), (ac), (ag), (am), (an), (aq), (as) und (be), wie unten definiert:
| | | | |
|---|---|---|---|
| -CH₂- | (aa) | 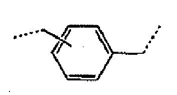 | (be) |
| -CH₂CH₂- | (ab) | | |
| -CH₂CH₂CH₂- | (ac) | | |
| | | | |
| -CH₂CH=CH- | (ag) | | |
| | | | |
| -C(=O)CH₂- | (am) | | |
| -C(=O)CH₂CH₂- | (an) | | |
| -CH₂C(=O)CH₂- | (aq) | | |
| -CR₂C(=O)C(CH₃)₂CH₂- | (as) | | |
bestehenden Gruppe ausgewählt ist.

10. Verbindungen nach einem der Ansprüche 1 bis 9, **dadurch gekennzeichnet, daß** Q² für einen aus der aus Wasserstoff, -NR¹R², Pir, -OR^{3a}, SR^{3b}, Aryl und Het bestehenden Gruppe ausgewählten Rest steht.

11. Verbindungen nach einem der Ansprüche 1 bis 10, **dadurch gekennzeichnet, daß** R¹ und R² jeweils unabhängig voneinander für einen aus der aus Wasserstoff, Alkyl und Alkyloxycarbonyl bestehenden Gruppe ausgewählten Rest stehen.

12. Verbindungen nach einem der Ansprüche 1 bis 10, **dadurch gekennzeichnet, daß** Pir für einen wenigstens einen Stickstoff enthaltenden Rest steht, der über den Stickstoff an den X²-Rest gebunden ist, ausgewählt aus der aus Piperidinyl und Isoindolyl bestehenden Gruppe; wobei die Pir-Reste jeweils gegebenenfalls durch 2 Oxoreste substituiert sind.

13. Verbindungen nach einem der Ansprüche 1 bis 10, **dadurch gekennzeichnet, daß** R^{3a} und R^{3b} jeweils unabhängig voneinander für einen Alkylrest stehen.

14. Verbindungen nach einem der Ansprüche 1 bis 10, **dadurch gekennzeichnet, daß** Het für einen aus der aus Pyridinyl, Furyl, Tetrahydropyranyl, Thienyl, Oxadiazolyl und Chinolinyl bestehenden Gruppe ausgewählten heterocyclischen Rest steht; wobei die Het-Reste jeweils gegebenenfalls durch einen oder mehrere aus der aus Halogen und Phenyl, gegebenenfalls substituiert durch (C₁₋₃)-Alkyloxy, bestehenden Gruppe ausgewählte Reste substituiert sind.

15. Verbindungen nach einem der Ansprüche 1 bis 10, **dadurch gekennzeichnet, daß** Aryl für Naphthyl oder Phenyl steht, die jeweils gegebenenfalls durch einen aus der aus (C₁₋₃)-Alkyl und Halogen bestehenden Gruppe ausgewählten Substituenten substituiert sind.

16. Verbindungen nach Anspruch 1, **dadurch gekennzeichnet, daß:**
A¹, A² jeweils unabhängig voneinander für ein Stickstoff- oder ein Kohlenstoffatom stehen; mit der Maßgabe, daß A¹ und A² nicht beide für ein Kohlenstoffatom stehen;
Z¹, Z² jeweils unabhängig voneinander für eine kovalente Bindung oder N-R⁴ stehen; wobei R⁴ aus der aus Wasserstoff und Aryl-(C₁₋₃)-alkyl bestehenden Gruppe ausgewählt ist;
n für eine ganze Zahl gleich Null, 1, 2 oder 3 steht;
R⁵ für Wasserstoff steht;
P für einen aus der aus Phenyl, Biphenyl, 1,1-Diphenylmethyl und Benzyloxyphenyl bestehenden Gruppe ausgewählten Rest steht;
X² für eine Bindung oder einen gesättigten oder ungesättigten (C₁₋₈)-Kohlenwasserstoffrest, wobei eine oder mehrere zweiwertige -CH₂-Einheiten gegebenenfalls durch eine entsprechende zweiwertige Phenyleinheit ersetzt sein können; und/oder wobei ein oder mehrere Wasserstoffatome durch einen Oxorest ersetzt sein können, steht;
Q² für einen aus der aus Wasserstoff, -NR¹R², Pir, -OR^{3a}, SR^{3b}, Aryl und Het bestehenden Gruppe ausgewählten Rest steht;
R¹ und R² jeweils unabhängig voneinander für einen aus der aus Wasserstoff, Alkyl und Alkyloxycarbonyl bestehenden Gruppe ausgewählten Rest stehen;
Pir für einen wenigstens einen Stickstoff enthaltenden Rest steht, der über den Stickstoff an den X²-Rest gebunden ist, ausgewählt aus der aus Piperidinyl und Isoindolyl bestehenden Gruppe; wobei die Pir-Reste jeweils gegebenenfalls durch 2 Oxoreste substituiert sind;
R^{3a}, R^{3b}, R^{3c} jeweils unabhängig voneinander für einen Alkylrest stehen;
Het für einen aus der aus Pyridinyl, Furyl, Tetrahydropyranyl, Thienyl, Oxadiazolyl und Chinolinyl bestehenden Gruppe ausgewählten heterocyclischen Rest steht; wobei die Het-Reste jeweils gegebenenfalls durch einen oder mehrere aus der aus Halogen und Phenyl, gegebenenfalls substituiert durch (C₁₋₃)-Alkyloxy, bestehenden Gruppe ausgewählte Reste substituiert sind; und
Aryl für Napthyl oder Phenyl, jeweils gegebenenfalls substituiert durch einen Substituenten, jeweils unabhängig voneinander ausgewählt aus der aus (C₁₋₃)-Alkyl und Halogen bestehenden Gruppe, steht.

17. Verbindungen nach einem der Ansprüche 1 bis 16 zur Verwendung als Medizin.

18. Pharmazeutische Zusammensetzung, enthaltend einen pharmazeutisch unbedenklichen Träger oder ein pharmazeutisch unbedenkliches Verdünnungsmittel und, als Wirkstoff, eine therapeutisch wirksame Menge einer Verbindung nach einem der Ansprüche 1 bis 16.

19. Pharmazeutische Zusammensetzung nach Anspruch 18, **dadurch gekennzeichnet, daß** sie weiterhin eine oder mehrere andere aus der aus Antidepressiva, Anxiolytika und Antipsychotika bestehenden Gruppe ausgewählte Verbindungen enthält.

20. Pharmazeutische Zusammensetzung nach Anspruch 18 oder 19, **dadurch gekennzeichnet, daß** sie in einer für die orale Verabreichung geeigneten Form vorliegt.

21. Verfahren zur Herstellung einer pharmazeutischen Zusammensetzung nach einem der Ansprüche 18 bis 20, **dadurch gekennzeichnet, daß** man einen pharmazeutisch unbedenklichen Träger innig mit einer therapeutisch wirksamen Menge einer Verbindung nach einem der Ansprüche 1 bis 16 mischt.

22. Verfahren zur Herstellung einer pharmazeutischen Zusammensetzung nach einem der Ansprüche 18 bis 20, **dadurch gekennzeichnet, daß** man einen pharmazeutisch unbedenklichen Träger innig mit einer therapeutisch wirksamen Menge einer Verbindung nach einem der Ansprüche 1 bis 16 und einer oder mehreren anderen aus der aus Antidepressiva, Anxiolytika und Antipsychotika bestehenden Gruppe ausgewählten Verbindungen mischt.

23. Verwendung einer Verbindung nach einem der Ansprüche 1 bis 16 zur Herstellung eines Medikaments zur Prävention und/oder Behandlung von Krankheiten, bei denen ein Antagonismus des α₂-adrenergen Rezeptors, insbesondere ein Antagonismus des α_{2C}-adrenergen Rezeptors, von therapeutischem Nutzen ist.

24. Verwendung einer Verbindung nach einem der Ansprüche 1 bis 16 zur Herstellung eines Medikaments zur Prävention und/oder Behandlung von Erkrankungen des zentralen Nervensystems, affektiven Psychosen, Angstzuständen, mit Streß in Zusammenhang stehenden Erkrankungen, die mit Depression und/oder Angstzuständen assoziiert sind, kognitiven Störungen, Persönlichkeitsstörungen, schizoaffektiven Störungen, Parkinson-Krankheit, Demenz des Alzheimer-Typs, chronischen Schmerzleiden, neurodegenerativen Krankheiten, Suchterkrankungen und sexueller Dysfunktion.

25. Verwendung einer Verbindung nach einem der Ansprüche 1 bis 16 in Kombination mit einem oder mehreren anderen aus der aus Antidepressiva, Anxiolytika und Antipsychotika bestehenden Gruppe ausgewählten Verbindungen zur Herstellung eines Medikaments zur Prävention und/oder Behandlung von Erkrankungen des zentralen Nervensystems, affektiven Psychosen, Angstzuständen, mit Streß in Zusammenhang stehenden Erkrankungen, die mit Depression und/oder Angstzuständen assoziiert sind, kognitiven Störungen, Persönlichkeitsstörungen, schizoaffektiven Störungen, Parkinson-Krankheit, Demenz des Alzheimer-Typs, chronischen Schmerzleiden, neurodegenerativen Krankheiten, Suchterkrankungen und sexueller Dysfunktion.

## Revendications

1. Composé selon la formule générale (I) 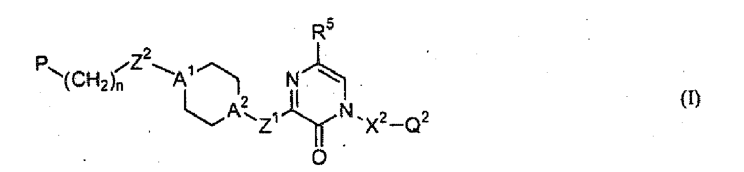 un sel d'addition d'acide ou de base pharmaceutiquement acceptable de celui-ci, une forme N-oxyde de celui-ci ou un sel d'ammonium quaternaire de celui-ci, dans laquelle :
A¹, A² sont chacun, indépendamment l'un de l'autre, un atome d'azote ou de carbone ; à condition que A¹ et A² ne soient pas simultanément un atome de carbone ;
Z¹, Z² sont chacun, indépendamment l'un de l'autre, une liaison covalente ou N-R⁴ ; où R⁴ est choisi dans le groupe d'hydrogène, (C₁₋₃)alkyle, aryle et aryl-(C₁₋₃)alkyle ;
n est un entier égal à zéro, 1, 2 ou 3 ;
R⁵ est choisi dans le groupe d'hydrogène ou halogéno ;
P est un radical choisi dans le groupe de phényle, biphényle, 1,1-diphénylméthyle et benzyloxyphényle ;
X² est une liaison covalente, un radical hydrocarboné en (C₁₋₈) saturé ou insaturé, où un ou plusieurs motifs -CH₂- bivalents peuvent être éventuellement remplacés par un motif phényle bivalent respectif ; et où un ou plusieurs atomes d'hydrogène peuvent être remplacés par un radical choisi dans le groupe d'oxo ; (C₁₋₃)alkyloxy ; halogéno ; cyano ; nitro ; formyle ; hydroxy ; amino ; trifluorométhyle ; mono- et di((C₁₋₃)alkyl)amino ; carboxy et thio ;
Q² est un radical choisi dans le groupe d'hydrogène ; -NR¹R² ; Pir ; -OR^{3a} ; SR^{3b} ; SO₂R^{3c} ; aryle ; et Het ; où deux radicaux -OR^{3a} peuvent être pris ensemble pour former un radical bivalent -O-(CH₂)ₛ-O- où s est un entier égal à 1, 2 ou 3 ;
R¹ et R² sont chacun, indépendamment l'un de l'autre, un radical choisi dans le groupe d'hydrogène ; alkyle ; alcényle ; alcynyle ; aryle ; arylalkyle ; diarylalkyle ; alkylcarbonyle ; alkylcarbonylalkyle ; alcénylcarbonyle ; alkyloxy ; alkyloxyalkyle ; alkyloxycarbonyle ; alkyloxyalkylcarbonyle ; alkyloxycarbonylalkyle ; alkyloxycarbonylalkylcarbonyle ; alkylsulfonyle ; arylsulfonyle ; arylalkylsulfonyle ; arylalcénylsulfonyle ; Hetsulfonyle ; arylcarbonyle ; aryloxyalkyle ; arylalkylcarbonyle ; Het ; Hetalkyle ; Het-alkylcarbonyle ; Hetcarbonyle ; Het-carbonylalkyle ; alkylNR^{a}R^{b} ; carbonyl-NR^{a}R^{b} ; carbonylalkylNR^{a}R^{b} ; alkylcarbonyl-NR^{a}R^{b} ; et alkylcarbonylalkyl-NR^{a}R^{b} ; où R^{a} et R^{b} sont choisis chacun indépendamment dans le groupe d'hydrogène, alkyle, alkylcarbonyle, alkyloxyalkyle, alkyloxycarbonylalkyle, aryle, arylalkyle, Het et alkyl-NR^{c}R^{d}, où R^{c} et R^{d} sont chacun indépendamment l'un de l'autre hydrogène ou alkyle ;
Pir est un radical contenant au moins un N, grâce auquel il est lié au radical X², choisi dans le groupe de pyrrolidinyle ; imidazolidinyle ; pyrazolidinyle ; pipéridinyle ; pipérazinyle ; pyrrolyle ; pyrrolinyle ; imidazolinyle ; pyrrazolinyle ; pyrrolyle ; imidazolyle ; pyrazolyle ; triazolyle ; azépyle ; diazépyle ; morpholinyle ; thiomorpholinyle ; indolyle ; isoindolyle ; indolinyle ; indazolyle ; benzimidazolyle ; et 1,2,3,4-tétrahydroisoquinoléinyle ; où chaque radical Pir est éventuellement substitué par 1, 2 ou 3 radicaux choisis dans le groupe d'hydroxy ; halogéno ; oxo ; (C₁₋₃)alkyle ; (C₁₋₃)alcényle ; (C₁₋₃)alkyloxycarbonyle ; Het-carbonyle ; (C₁₋₃)alkylamino ; trifluorométhyle ; phényl(C₀₋₃)alkyle ; pyrimidinyle ; pyrrolidinyle ; et pyridinyloxy ;
R^{3a} est un radical choisi dans le groupe d'hydrogène ; alkyle ; trihalogénoalkyle ; arylalkyle ; alkyloxyalkyle ; Het ; et Het-alkyle ;
R^{3b}, R^{3c} sont chacun, indépendamment l'un de l'autre, un radical choisi dans le groupe d'hydrogène ; alkyle ; trihalogénoalkyle ; aryle ; arylalkyl alkyloxyalkyle ; Het ; et Het-alkyle ;
Het est un radical hétérocyclique choisi dans le groupe de pyrrolidinyle ; imidazolidinyle ; pyrazolidinyle ; pipéridinyle ; pipérazinyle ; pyrrolyle ; pyrrolinyle ; imidazolinyle ; pyrrazolinyle ; pyrrolyle ; imidazolyle ; pyrazolyle ; triazolyle ; pyridinyle ; pyridazinyle ; pyrimidinyle ; pyrazinyle ; triazinyle ; azépyle ; diazépyle ; morpholinyle ; thiomorpholinyle ; indolyle ; isoindolyle ; indolinyle ; indazolyle ; benzimidazolyle ; 1,2,3,4-tétrahydroisoquinoléinyle ; furyle ; tétrahydropyranyle ; thiényle ; oxazolyle ; isoxazolyle ; thiazolyle ; thiadiazolyle ; isothiazolyle ; dioxolyle ; dithianyle ; tétrahydrofuryle ; tétrahydropyranyle ; oxadiazolyle ; quinoléinyle ; isoquinoléinyle ; quinoxalinyle ; benzoxazolyle ; benzisoxazolyle ; benzothiazolyle ; benzisothiazolyle ; benzofuranyle ; benzothiényle ; benzopipéridinyle ; benzomorpholinyle ; chroményle ; et imidazo[1,2-α]pyridinyle ; où chaque radical Het est éventuellement substitué par un ou plusieurs radicaux choisis dans le groupe d'halogéno ; oxo ; (C₁₋₃)alkyle ; phényle, éventuellement substitué par (C₁₋₃)alkyloxy, (C₁₋₃)alkylcarbonyle ; (C₁₋₃)alcénylthio ; imidazolyl-(C₁₋₃)alkyle ; aryl (C₁₋₃)alkyle et (C₁₋₃)alkyloxycarbonyle ;
aryle est naphtyle ou phényle, substitué chacun éventuellement par 1, 2 ou 3 substituants choisis chacun indépendamment les uns des autres dans le groupe d'oxo ; (C₁₋₃)alkyle ; (C₁₋₃)alkyloxy ; halogéno ; cyano ; nitro ; formyle ; éthanoyle ; hydroxy ; amino ; trifluorométhyle ; mono- et di((C₁₋₃)alkyl) amino ; mono- et di((C₁₋₃)alkylcarbonyl) amino ; carboxy ; morpholinyle ; et thio ;
alkyle est, sauf indication contraire, un radical hydrocarboné saturé droit ou ramifié ayant de 1 à 8 atomes de carbone ; ou un radical hydrocarboné saturé cyclique ayant de 3 à 7 atomes de carbone ; ou un radical hydrocarboné saturé cyclique ayant de 3 à 7 atomes de carbone lié à un radical hydrocarboné saturé droit ou ramifié ayant de 1 à 8 atomes de carbone ; où chaque radical est éventuellement substitué sur un ou plusieurs atomes de carbone par un ou plusieurs radicaux choisis dans le groupe d'oxo ; (C₁₋₃)alkyloxy ; halogéno ; cyano ; nitro ; formyle ; hydroxy ; amino ; carboxy ; et thio ;
alcényle est un radical alkyle tel que défini ci-dessus, ayant en outre une ou plusieurs doubles liaisons ;
alcynyle est un radical alkyle tel que défini ci-dessus, ayant en outre une ou plusieurs triples liaisons ;
arylalkyle est un radical alkyle tel que défini ci-dessus, ayant en outre un groupement CH₃ remplacé par phényle ; et
diarylalkyle est un radical alkyle tel que défini ci-dessus, ayant en outre deux groupements CH₃ remplacés par phényle.

2. Composé selon la revendication 1, **caractérisé en ce que** le motif 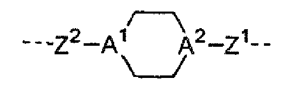 est un radical bivalent de formule (II-a), (II-b), (II-c) ou (II-d), tel que présenté ci-dessous.

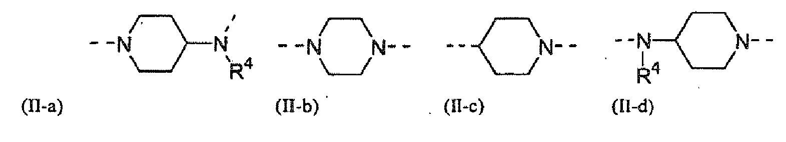

3. Composé selon la revendication 2, **caractérisé en ce que** R⁴ est hydrogène ou p-aminométhylbenzyle.

4. Composé selon l'une quelconque des revendications 1 à 3, **caractérisé en ce que** n est 1, 2 ou 3.

5. Composé selon l'une quelconque des revendications 1 à 4, **caractérisé en ce que** R⁵ est hydrogène.

6. Composé selon l'une quelconque des revendications 1 à 5, **caractérisé en ce que** P est phényle.

7. Composé selon l'une quelconque des revendications 1 à 6, **caractérisé en ce que** X² est choisi dans le groupe d'une liaison covalente, d'un radical hydrocarboné en C₁, d'un radical hydrocarboné en C₂ ou d'un radical hydrocarboné en C₃.

8. Composé selon l'une quelconque des revendications 1 à 7, **caractérisé en ce qu'**un motif bivalent-CH₂- du radical hydrocarboné X² est remplacé par un motif phényle bivalent ; ou **en ce que** deux atomes d'hydrogène du radical hydrocarboné X² sont remplacés par un radical oxo.

9. Composé selon l'une quelconque des revendications 1 à 8, **caractérisé en ce que** X² est choisi dans le groupe d'une liaison covalente et d'un quelconque des radicaux (aa), (ab), (ac), (ag), (am), (an), (aq), (as) et (be) tels que définis ci-dessous :
| | | | |
|---|---|---|---|
| -CH₂- | (aa) | 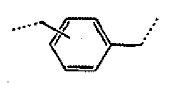 | (be) |
| -CH₂CH₂- | (ab) | | |
| -CH₂CH₂CH₂- | (ac) | | |
| | | | |
| -CH₂CH=CH- | (ag) | | |
| | | | |
| -C(=O)CH₂- | (am) | | |
| -C(=O)CH₂CH₂- | (an) | | |
| -CH₂C(=O)CH₂- | (aq) | | |
| -CH₂C(=O)C(CH₃)₂CH₂- | (as) | | |

10. Composé selon l'une quelconque des revendications 1 à 9, **caractérisé en ce que** Q² est un radical choisi dans le groupe d'hydrogène ; -NR¹R² ; Pir ; -OR^{3a} ; SR^{3b} ; aryle ; et Het.

11. Composé selon l'une quelconque des revendications 1 à 10, **caractérisé en ce que** R¹ et R² sont chacun, indépendamment l'un de l'autre, un radical choisi dans le groupe d'hydrogène ; alkyle ; et alkyloxycarbonyle.

12. Composé selon l'une quelconque des revendications 1 à 10, **caractérisé en ce que** Pir est un radical contenant au moins un N, grâce auquel il est lié au radical X², choisi dans le groupe de pipéridinyle et isoindolyle ; chaque radical Pir étant éventuellement substitué par 2 radicaux oxo.

13. Composé selon l'une quelconque des revendications 1 à 10, **caractérisé en ce que** R^{3a} et R^{3b} sont chacun, indépendamment l'un de l'autre, un radical alkyle.

14. Composé selon l'une quelconque des revendications 1 à 10, **caractérisé en ce que** Het est un radical hétérocyclique choisi dans le groupe de pyridinyle ; furyle ; tétrahydropyranyle ; thiényle ; oxadiazolyle ; et quinoléinyle ; chaque radical Het étant éventuellement substitué par un ou plusieurs radicaux choisis dans le groupe d'halogéno et phényle, éventuellement substitués par (C₁₋₃)alkyloxy.

15. Composé selon l'une quelconque des revendications 1 à 10, **caractérisé en ce qu'**aryle est naphtyle ou phényle, chacun éventuellement substitué par un substituant choisi dans le groupe de (C₁₋₃)alkyle et halogéno.

16. Composé selon la revendication 1, **caractérisé en ce que :**
A¹, A² sont chacun, indépendamment l'un de l'autre, un atome d'azote ou de carbone ; à condition que A¹ et A² ne soient pas simultanément un atome de carbone ;
Z¹, Z² sont chacun, indépendamment l'un de l'autre, une liaison covalente ou N-R⁴ ; où R⁴ est choisi dans le groupe d'hydrogène et aryl-(C₁₋₃)alkyle ;
n est un entier égal à zéro, 1, 2 ou 3 ;
R⁵ est hydrogène ;
P est un radical choisi dans le groupe de phényle, biphényle, 1,1-diphénylméthyle et benzyloxyphényle ;
X² est une liaison, un radical hydrocarboné en (C₁₋₈) saturé ou insaturé, où un ou plusieurs motifs-CH₂- bivalents peuvent éventuellement être remplacés par un motif phényle bivalent respectif ; et/ou où un ou plusieurs atomes d'hydrogène peuvent être remplacés par un radical oxo ;
Q² est un radical choisi dans le groupe d'hydrogène ; -NR¹R² ; Pir ; -OR^{3a} ; SR^{3b} ; aryle ; et Het ;
R¹ et R² sont chacun, indépendamment l'un de l'autre, un radical choisi dans le groupe d'hydrogène ; alkyle ; et alkyloxycarbonyle ;
Pir est un radical contenant au moins un N, grâce auquel il est lié au radical X², choisi dans le groupe de pipéridinyle ; isoindolyle ; où chaque radical Pir est éventuellement substitué par 2 radicaux oxo ;
R^{3a}, R^{3b}, R^{3c} sont chacun, indépendamment les uns des autres, un radical alkyle ;
Het est un radical hétérocyclique choisi dans le groupe de pyridinyle ; furyle ; tétrahydropyranyle ; thiényle ; oxadiazolyle ; et quinoléinyle ; où chaque radical Het est éventuellement substitué par un ou plusieurs radicaux choisis dans le groupe d'halogéno ; et phényle, éventuellement substitué par (C₁₋₃)alkyloxy ; et
aryle est naphtyle ou phényle, chacun éventuellement substitué par un substituant, chacun indépendamment l'un de l'autre, choisi dans le groupe de (C₁₋₃)alkyle et halogéno.

17. Composé selon l'une quelconque des revendications 1 à 16, destiné à être utilisé comme médicament.

18. Composition pharmaceutique comprenant un support ou un diluant pharmaceutiquement acceptable et, à titre de principe actif, une quantité thérapeutiquement efficace d'un composé selon l'une quelconque des revendications 1 à 16.

19. Composition pharmaceutique selon la revendication 18, **caractérisée en ce qu'**elle comprend en outre un ou plusieurs autres composés choisis dans le groupe des antidépresseurs, anxiolytiques et antipsychotiques.

20. Composition pharmaceutique selon l'une ou l'autre des revendications 18 et 19, **caractérisée en ce qu'**elle est sous une forme adaptée à l'administration par voie orale.

21. Procédé de préparation d'une composition pharmaceutique selon l'une quelconque des revendications 18 à 20, **caractérisé en ce qu'**un support pharmaceutiquement acceptable est mélangé intimement avec une quantité thérapeutiquement efficace d'un composé selon l'une quelconque des revendications 1 à 16.

22. Procédé de préparation d'une composition pharmaceutique selon l'une quelconque des revendications 18 à 20, **caractérisé en ce qu'**un support pharmaceutiquement acceptable est mélangé intimement avec une quantité thérapeutiquement efficace d'un composé selon l'une quelconque des revendications 1 à 16 et un ou plusieurs autres composés choisis dans le groupe des antidépresseurs, anxiolytiques et antipsychotiques.

23. Utilisation d'un composé selon l'une quelconque des revendications 1 à 16, pour la préparation d'un médicament destiné à la prévention et/ou traitement de maladies pour lesquelles l'antagonisme du récepteur α₂-adrénergique, en particulier l'antagonisme du récepteur α_{2C}-adrénergique est thérapeutiquement utile.

24. Utilisation d'un composé selon l'une quelconque des revendications 1 à 16, pour la préparation d'un médicament destiné à la prévention et/ou traitement des troubles du système nerveux central, des troubles d'humeur, des troubles d'anxiété, des troubles liés au stress et associés à la dépression et/ou à l'anxiété, des troubles cognitifs, des troubles de personnalité, des troubles schizoaffectifs, de la maladie de Parkinson, de la démence de type Alzheimer, des affections de douleur chronique, des maladies neurodégénératives, des troubles d'accoutumance et du dysfonctionnement sexuel.

25. Utilisation d'un composé selon l'une quelconque des revendications 1 à 16, en combinaison avec un ou plusieurs autres composés choisis dans le groupe des antidépresseurs, anxiolytiques et antipsychotiques pour la préparation d'un médicament destiné à la prévention et/ou traitement des troubles du système nerveux central, des troubles d'humeur, des troubles d'anxiété, des troubles liés au stress et associés à la dépression et/ou à l'anxiété, des troubles cognitifs, des troubles de personnalité, des troubles schizoaffectifs, de la maladie de Parkinson, de la démence de type Alzheimer, des affections de douleur chronique, des maladies neurodégénératives, des troubles d'accoutumance et du dysfonctionnement sexuel.
